# EUROPEAN PATENT APPLICATION

(11) **EP 1 834 636 A1**
(43) Date of publication of application: **19.09.2007**
(21) Application number: 07103789.9
(22) Date of filing: 08.03.2007
(51) Int. Cl.: A61K 9/51, A61L 27/56, A61L 29/14, A61L 31/14, A61L 31/10, A61K 31/337, A61K 31/352, A61K 31/436, A61K 31/727

(54) **Compositions comprising porous articles and uses in implantable medical devices**

(30) Priority: 08.03.2006 US 780121 P; 20.06.2006 US 814973 P; 21.07.2006 US 832383 P; 20.10.2006 US 862263 P; 20.10.2006 US 862265 P; 20.10.2006 US 862270 P
(71) Applicant: Sahajanand Medical Technologies PVT. ltd, Saiyedpura, Surat 395003 GUJ (IN)
(72) Inventor: Patravale, Vandana B., Andheri-Kurla Road, 400 093, Mumbai (IN); Kothwala, Devesh, 395 0003, Surat (IN); Raval, Ankur J., 395 009, Adajan, Surat (Gujarat) (IN); Kotadia, Haresh, 395 003, Saiyedpura, Surat (Gujarat) (IN)
(74) Representative: Swinkels, Bart Willem

(57) **Abstract**

The present invention relates to a composition comprising a first polymer having pores, nanoparticles dispersed within the pores of the first polymer, the nanoparticles comprising a second polymer and at least one pharmaceutically active agent dispersed in the second polymer; and heparin covalently bonded to at least one of the first and second polymer. The invention further relates to medical devices coated with the composition and uses thereof.
In a preferred embodiment, the invention relates to a stent coated with porous heparinized poly-l-lactic acid or heparinized PVP. Active agents are chosen from paclitaxel, rapamycin, genistein, flavonoid, LY294002 or LY303511 (both benzopyran-4-one compounds).

## Description

### FIELD OF THE INVENTION

The present invention relates to stents coated with nanoporous articles comprising nanoparticles comprising at least one pharmaceutically active agent and at least one polymer.

### BACKGROUND OF THE INVENTION

The human or animal body comprises many passageways for transport of essential materials. These include, for example, the vascular system for transport of blood, the various passageways of the gastrointestinal tract, the urinary tract, the airways, as well as the reproductive tracts. Various insults to these passageways (e.g., injury, surgical procedures, inflammation or neoplasms) can produce narrowing or even obstruction of such body passageways, with serious consequences that may ultimately result in death.

One approach to the problem of narrowing or obstructed body passageways has been the insertion of endoluminal stents. Stents are devices having a generally tubular structure that are placed into the lumen of a body passageway to physically hold open a passageway that is narrowed or blocked by, e.g., a tumor or other tissues/substances/pathological processes like occlusive atherothrombosis. Frequently, however, the body responds to the implanted stent by ingrowth into the lumen of the stent, thereby again narrowing or blocking the passageway into which the stent was placed. In the case of stents that are used in the context of a neoplastic obstruction, the tumor is usually able to grow into the lumen of the stent. In non-neoplastic settings, the presence of a stent in the lumen of a body passageway can induce the ingrowth of reactive or inflammatory tissue (e.g., blood vessels, fibroblasts and white blood cells) into lumen of the stent. In a vascular disease setting, restenosis subsequent to balloon angioplasty (with or without stenting) can occur. Multiple processes, including thrombosis, inflammation, growth factor and cytokine release, cell proliferation, cell migration and extracellular matrix synthesis may each contribute to the restenotic process. Upon pressure expansion of an intracoronary balloon catheter during angioplasty, both endothelial and smooth muscle cells within the vessel wall become injured, initiating proliferative, thrombotic and inflammatory responses that ultimately can lead to occlusion of the implanted stent.

Thrombosis is a prime concern after an implant of a drug eluting stent. Various anticoagulants have been systemically used orally and/or intravenously to overcome or to minimize the risk of thrombus formation at the stented region and in the immediate neighborhood of the stent. However, bleeding and other complications may occur from the use of aggressive treatments such as anticoagulants (e.g., clopidogrel, LMW, heparin, ticlopidine, aspirin or any other GP II_{b}/IIIₐ inhibitors).

Accordingly, there remains a need for a more localized delivery of anticoagulants and other pharmaceutically active agents.

### SUMMARY OF THE INVENTION

One embodiment provides a composition comprising:
a first polymer having pores;
nanoparticles dispersed within the pores of the first polymer, the nanoparticles comprising a second polymer and at least one pharmaceutically active agent dispersed in the second polymer; and
heparin covalently bonded to at least one of the first and second polymer.

In one embodiment, the heparin is covalently bonded to one or both of the first and second polymers.

In one embodiment, the polymer covalently bonded to heparin is biodegradable.

In one embodiment, one or both of the first and second polymers is biodegradable.

In one embodiment, the biodegradable polymer is chosen from polylactides.

In one embodiment, the biodegradable polymer is chosen from poly(1-lactide), racemic polylactide, poly(1-lactide-co-glycolide), racemic poly(1-lactide-co-glycolide), poly(1-lactide-co-caprolactone poly(d,1-lactide-co-caprolactone), poly(1-lactide-co-trimethylene carbonate) and poly(d,1-lactide-co-trimethylene carbonate).

In one embodiment, the first and second polymers are the same.

In one embodiment, at least one of the first and second polymers comprises a blend of one or more polymers.

Another embodiment provides a device comprising a coating for at least a portion thereof, the coating comprising a composition comprising:
a first polymer having pores;
nanoparticles dispersed within the pores of the first polymer, the nanoparticles comprising a second polymer and at least one pharmaceutically active agent dispersed in the second polymer; and
heparin covalently bonded to at least one of the first and second polymers.

In one embodiment, the device is selected from sutures, staples, anastomosis devices, vertebral disks, bone pins, suture anchors, hemostatic barriers, clamps, screws, plates, clips, vascular implants, tissue scaffolds, bone substitutes, intraluminal devices, and vascular supports.

In one embodiment, the device is implantable into a mammalian lumen.

In one embodiment, the device is a stent.

In one embodiment, the concentration of the at least one pharmaceutically active agent based on the surface area of the stent ranges from 0.1 to about 5 µg/mm².

In one embodiment, the concentration of the at least one pharmaceutically active agent based on the surface area of the stent ranges from about 0.7 µg/mm² to about 3.0 µg/mm²

In one embodiment, the concentration of the at least one pharmaceutically active agent based on the surface area of the stent ranges from about 1.0 to about 1.8 µg/mm²

In one embodiment, the concentration of the at least one pharmaceutically active agent based on the surface area of the stent ranges from about 1.0 to about 1.4 µg/mm².

In one embodiment, the coating contacts the medical device.

In one embodiment, the coating contacts at least one inner coating that contacts the medical device.

In one embodiment, the at least one inner coating is chosen from ceramics, nonbiodegradable polymers, metals, and carbon.

In one embodiment, the device further comprises a protective coating over the coating, wherein the protective coating is free of a pharmaceutically active agent.

In one embodiment, the protective coating comprises at least one biodegradable polymer.

In one embodiment, the at least one biodegradable polymer is covalently bonded to heparin.

In one embodiment, the device further comprises at least one additional coating comprising a polymer covalently bonded to heparin, the at least one additional coating comprising at least one pharmaceutically active agent. In one embodiment, wherein the polymer is biodegradable. In one embodiment, the device comprises at least two additional coatings. In one embodiment, the device comprises at least one additional coating and a protective coating.

In one embodiment, at least one of the first and second polymers degrades by hydrolysis in a natural intraluminal human body environment at preselected rates of degradation.

In one embodiment, the at least one pharmaceutically active agent is chosen from antithrombotics, anticoagulants, antiplatelet agents, thrombolytics, antiproliferatives, anti-inflammatories, antimitotic, antimicrobial, agents that inhibit restenosis, smooth muscle cell inhibitors, antibiotics, fibrinolytic, immunosuppressive, and anti-antigenic agents.

In one embodiment, the at least one pharmaceutically active agent is chosen from paclitaxel, sirolimus, flavonoids, compounds of formula A 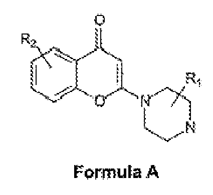 wherein R1 and R2 are each independently selected from alkyl, substituted alkyl, heteroalkyl, cycloalkyl, substituted cycloalkyl, heterocycloalkyl, substituted heterocycloalkyl, aryl, substituted aryl, heteroaryl, substituted heteroaryl, alkoxy, halogen, hydroxy, and amino,
and compounds of formula B, 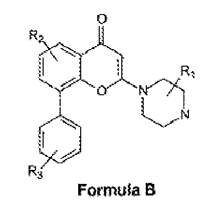 wherein the presence of each of R1 and R2 and R3 is optional and R1 and R2 and R3 are each independently selected from alkyl, aryl, alkoxy, halogen, hydroxy, and amino.

In one embodiment, the flavonoid is selected from chalcones, dihydrochalcones, flavanones, flavonols, dihydroflavonols, flavones, flavanols, isoflavones, neoflavones, aurones, anthocyanidins, proanthocyanidins and isoflavanes.

In one embodiment, the flavonoid is selected from flavanones, flavonols, and isoflavones.

In one embodiment, the flavonoid is selected from narigenin, naringin, eriodictyol, hesperetin, hesperidin (esperidine), kampferol, quercetin, rutin, cyanidol, meciadonol, catechin, epi-gallocatechin-gallate, taxifolin (dihydroquercetin), genistein, genistin, daidzein, biochanin, glycitein, chrysin, diosmin, luetolin, apigenin, tangeritin and nobiletin.

In one embodiment, the at least one pharmaceutically active agent is paclitaxel.

In one embodiment, the at least one pharmaceutically active agent is sirolimus.

In one embodiment, the at least one pharmaceutically active agent is genistein.

In one embodiment, at least one of the first and second polymers degrade by hydrolysis in a natural intraluminal human body environment at preselected rates of degradation.

Another embodiment provides a device comprising a coating, comprising:
a polymer having pores;
nanoparticles dispersed within the pores of the polymer, the nanoparticles comprising at least one pharmaceutically active agent;
wherein the at least one pharmaceutically active agent is chosen from composition comprising at least one pharmaceutically active agent selected from genistein or compounds having the structure of Formula A and Formula B below: 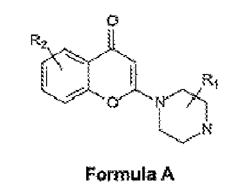 wherein R1 and R2 are each independently selected from alkyl, substituted alkyl, heteroalkyl, cycloalkyl, substituted cycloalkyl, heterocycloalkyl, substituted heterocycloalkyl, aryl, substituted aryl, heteroaryl, substituted heteroaryl, alkoxy, halogen, hydroxy, and amino, 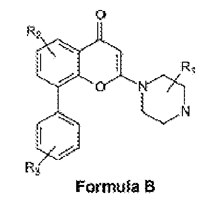 wherein the presence of each of R1 and R2 and R3 is optional and R1 and R2 and R3 are each independently selected from alkyl, aryl, alkoxy, halogen, hydroxy, and amino.

In one embodiment, the nanoparticles further comprise a polymer that is the same or different from the porous polymer, the nanoparticles being porous or nonporous.

In one embodiment, at least one of the nanoparticulate polymer and the porous polymer is biodegradable.

In one embodiment, the biodegradable polymer is chosen from poly(1-lactide), racemic polylactide, poly(l-lactide-co-glycolide), racemic poly(1-lactide-co-glycolide), poly(1-lactide-co-caprolactone poly(d,l-lactide-co-caprolactone), poly(1-lactide-co-trimethylene carbonate) and poly(d,l-lactide-co-trimethylene carbonate).

In one embodiment, at least one of the nanoparticulate polymer and the porous polymer further comprises heparin.

In one embodiment, the heparin is covalently bonded to the polymer.

In one embodiment, both the nanoparticulate polymer and the porous polymer comprise a biodegradable polymer covalently bound to heparin

Another embodiment provides a method of making an implantable medical device comprising:
coating the implantable medical device with a mixture comprising:
   a polymer, polymer suspension, or polymer-containing solution, and
   a solvent;
changing the temperature of the mixture to induce a phase separation of a polymer-rich phase and a polymer-poor phase; and
removing the solvent to form a porous polymer coating on the device.

In one embodiment, the method further comprises impregnating the pores of the polymer with nanoparticles comprising at least one pharmaceutically active agent. In one embodiment, the nanoparticles further comprise a polymer which may be the same or different as the porous polymer. In one embodiment, at least one of the porous polymer or the nanoparticulate polymer is covalently bound to heparin. In one embodiment, at least one of the porous polymer or the nanoparticulate polymer is biodegradable. In one embodiment, at least one of the porous polymer or the nanoparticulate polymer comprises one or more of poly(l-lactide), racemic polylactide, poly(l-lactide-co-glycolide), racemic poly(1-lactide-co-glycolide), poly(1-lactide-co-caprolactone poly(d,l-lactide-co-caprolactone), poly(1-lactide-co-trimethylene carbonate) and poly(d,1-lactide-co-trimethylene carbonate). In one embodiment, both the nanoparticulate polymer and the porous polymer comprises a biodegradable polymer covalently bonded to heparin.

Another embodiment provides a method of treating at least one disease or condition associated with vascular injury or angioplasty comprising, implanting in a subject in need thereof a medical device having a coating for at least a portion thereof comprising a composition comprising:
a first polymer having pores;
nanoparticles dispersed within the pores of the first polymer, the nanoparticles comprising a second polymer and at least one pharmaceutically active agent dispersed in the second polymer; and
heparin covalently bonded to at least one of the first and second polymer.

In one embodiment, the heparin is covalently bonded to both the first and second polymers.

In one embodiment, the polymer covalently bonded to heparin is biodegradable.

In one embodiment, one or both of the first and second polymers is biodegradable.

In one embodiment, the biodegradable polymer is chosen from polylactides.

In one embodiment, the biodegradable polymer is chosen from poly(l-lactide), racemic polylactide, poly(1-lactide-co-glycolide), racemic poly(l-lactide-co-glycolide), poly(1-lactide-co-caprolactone poly(d,l-lactide-co-caprolactone), poly(1-lactide-co-trimethylene carbonate) and poly(d,l-lactide-co-trimethylene carbonate).

In one embodiment, the first and second polymers are the same.

In one embodiment, at least one of the first and second polymers comprises a blend of one or more polymers.

In one embodiment, the at least one disease or condition is a proliferative disorder.

In one embodiment, the proliferative disorder is restenosis.

In one embodiment, the proliferative disorder is a tumor.

In one embodiment, the proliferative disorder comprises the proliferation of smooth muscle cells.

In one embodiment, the at least one disease or condition is an inflammatory disease.

In one embodiment, the at least one disease or condition is an autoimmune disease.

In one embodiment, the at least one disease or condition is neointima and neointimal hyperplasia.

In one embodiment, the at least one disease or condition is selected from thrombosis, embolism, and platelet accumulation.

### BRIEF DESCRIPTION OF THE DRAWINGS

Various embodiments of the invention will be understood from the following description, the appended claims and the accompanying drawings, in which:

FIG. 1 is a visual schematic of an embodiment of a biological mechanism by which heparin acts;

FIG. 2 is a schematic ofthe action ofsirolimus on cell division; and

FIG. 3 is an experimental set-up for a heparinization process for poly(l-lactide).

### DETAILED DESCRIPTION

The present invention relates to implantable medical devices having coatings comprising porous polymers and nanoparticles comprising pharmaceutically active agents for controlled release of the agents.

One embodiment ofthe invention provides a composition comprising:
a first polymer having pores;
nanoparticles dispersed within the pores of the first polymer, the nanoparticles comprising a second polymer and at least one pharmaceutically active agent dispersed in the second polymer; and
heparin covalently bonded to at least one of the first and second polymer.

Polymers having pores are well known in the art and methods for preparing porous polymers are encompassed by the invention, including foaming, mixing with gas, curing or setting in high humidity, etc.

In one embodiment, the porous polymer, is prepared with the aid of a porogen. A porogen is an additive combined with either a polymeric material or precursors to a polymeric material. Removal of the porogen, accompanied by the step of curing or setting the polymer if necessary, results in pores in the region once occupied by the porogen.

Exemplary porogens include salts (e.g., sodium bicarbonate), gelatins, sugars (e.g., glucose, sucrose, mannitol), polymeric particles (e.g., polyalkylene glycols, polysaccharides), solvents (e.g., water, alcohols, organic solvents), small organic molecules that are soluble in solvents (e.g., urea, citric acid, ascorbic acid, vitamin E, small chain hydrophilic molecules), frozen carbon dioxide, and combinations thereof. Porogens can be removed by allowing a solution to warm, e.g., in the case of frozen carbon dioxide, or by adding a solvent that dissolves the porogen but not the polymer.

Porous polymers that encapsulate nanoparticles comprising pharmaceutically active agents may release the agents faster than normal porous polymers, if desired. The release rate can depend at least in part on the pore size, which in turn can depend on the size of the porogen. For example, larger pores can result in faster release rates compared to smaller pores. The size of the porogen can be selected to ultimately tailor the pore size in the porous polymer. In one embodiment, the pores in the porous polymer have at least one mean dimension that ranges from about 0.01 µm to about 500 µm, from about 0.01 µm to about 200 µm, from about 0.01 µm to about 100 µm, from about 0.01 µm to about 50 µm, from about 0.1 µm to about 500 µm, from about 0.1 µm to about 200 µm, from about 0.1 µm to about 100 µm, from about 0.1 µm to about 50 µm, from about 1 µm to about 500µm, from about 1 µm to about 200 µm, of from about 1 µm to about 100 µm, or from about 0.1 µm to about 50 µm. Other pore size ranges can be readily envisioned and accessed by the choice of the porogen and reaction conditions. In another embodiment, pores have at least two mean dimensions that fall within the disclosed ranges.

In one embodiment, the porogens are removed by particle leaching. In this method, a mixture containing the porogen and polymer (or polymer precursors) are subjected to a solvent, such as an organic solvent or water, that dissolves the porogen only. The porogen can be washed away with the solvent, leaving the polymer with pores.

In another embodiment, the porogens are removed by thermally induced phase separation (TIPS). In this method, solvent is used as a pore-generating solvent and is combined with the polymer (or polymer precursor) to form a homogenous mixture. The temperature is altered in a sufficient amount (by either raising or lowering the temperature) to cause a phase separation between a polymer-rich phase and a polymer-poor phase. In one embodiment where a phase separation is caused by lowering the temperature, quenching the polymer solution below the freezing point of the solvent and subsequently freeze dried to remove the solvent. In another embodiment, phase separation can be obtained by raising the temperature and concurrently or subsequently removing the solvent by evaporation, optionally with the aid of a vacuum. Exemplary methods for performing TIPS can be found in Y.S. Nam and T.G. Park, "Porous biodegradable polymeric scaffolds prepared by thermally induced phase separation," J. Biomed. Mater. Res. 47:8-17, 1999, the disclosure of which is incorporated herein by reference.

In another embodiment, porous articles can be prepared by the use of solid particle additives, as described in U.S. Patent Publication No. 20060088567, the disclosure of which is incorporated herein by reference. Solid particle additives are lodged in regions of the polymer and create a network of interconnecting pores. The pore size can be controlled by the size of the solid particle additives.

In one embodiment, the nanoparticles have a mean diameter ranging from about 0.1 nm to about 1 micron. Other mean diameter ranges less than 1 micron can be easily envisioned. The nanoparticles can be made by any method known in the art, such as those methods disclosed in U.S. Patent No. 5,716,981, the disclosure of which is incorporated herein by reference

Either or both of the porous polymer or the nanoparticulate polymers can further comprise heparin that is covalently bonded to the polymer. Heparin is an anionic, multi-sulfate, mucopolysaccharide used as an anticoagulant. Heparin acts as an anticoagulant by binding to antithrombin III and inhibiting thrombogenesis primarily through inactivation of factors, IIa and Xa. In one embodiment, heparin can achieve at least one of the following functions, including inhibiting the activation of coagulation, potentiating the inhibition of the activated coagulation enzymes, and preventing platelet adhesion to the surface ofthe device.

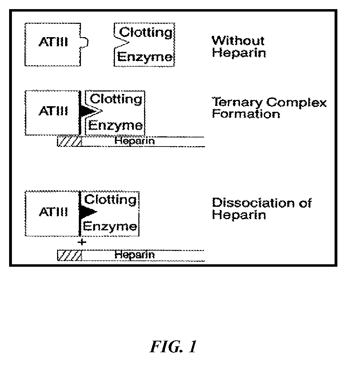

Heparin has also been used as a systemic anti-coagulant in humans and in connection with stent coatings for local delivery for prevention of stent related thrombotic events. Accumulation of platelets at portions of an implanted stent remains a drawback that affects clinical safety and efficacy. Heparin has been evaluated as a stent coating for preventing early and late thrombosis and found to be satisfactory to inhibit sub acute thrombosis (SAT). The morphology of blood vessels observed after coronary stenting demonstrates that thrombus occurs at an early stage in addition to acute inflammation (proliferation and migration of smooth muscle cells) followed by neointimal growth. The occurrence of increased inflammation soon after stenting is associated with medial injury and lipid core penetration by stent struts. FIG. 1 shows a visual schematic of an embodiment of a biological mechanism by which heparin acts.

The present invention provides a polymer covalently bonded to heparin, also referred to herein as a "heparinized polymer." The present invention also relates to heparinized polymers, such as heparinized biodegradable polymers, combined with at least one pharmaceutically active agent, such as sirolimus (rapamycin), paclitaxel or flavonoids, or mixtures of two or more of the foregoing select drugs. Another embodiment relates to coating on coronary stents comprising the heparinized polymer compositions, which can be used, for example, for preventing early platelet adhesion and excessive smooth muscle cell (SMC) proliferation. Heparin, covalently coupled to the coating polymer, may be released throughout the entire period of biological degradation of the coating polymer thus maximizing the biocompatibility of the coated stent.

In one embodiment, the polymer is biodegradable. "Biodegradable polymer," as used herein, refers to a polymer capable of decomposing, degenerating, degrading, depolymerizing, or any other mechanism that reduces the molecular weight of the polymer. In one embodiment, the resulting product(s) of biodegradation is soluble in the resulting body fluid or, if insoluble, can be suspended in a body fluid and transported away from the implantation site without clogging the flow of the body fluid. The body fluid can be any fluid in the body of a mammal including, but not limited to, blood, urine, saliva, lymph, plasma, gastric, biliary, or intestinal fluids, seminal fluids, and mucosal fluids or humors. In one embodiment, the biodegradable polymer is soluble, degradable as defined above, or is an aggregate of soluble and/or degradable material(s) with insoluble material(s) such that, with the resorption of the soluble and/or degradable materials, the residual insoluble materials are of sufficiently fine size such that they can be suspended in a body fluid and transported away from the implantation site without clogging the flow of the body fluid. Ultimately, the degraded compounds are eliminated from the body either by excretion in perspiration, urine or feces, or dissolved, degraded, corroded or otherwise metabolized into soluble components that are then excreted from the body.

In one embodiment, the use of biodegradable polymers can ensure that the therapeutic agents and the polymer cease to exist in the vessel wall after a predetermined period. In one embodiment, the predetermined period is 48-55 days after implantation. In the context of vascular angioplasty the devices of the invention can deprive the vascular elements a nidus to initiate a cascade of detrimental secondary effects.

Suitable biodegradable polymers include poly(ethylene vinyl acetate), polyanhydrides, polyglycolic acid, collagen, polyorthoesters, polyesters, polyalkylcyanoacrylates, polyorthoesters, polyanhydrides, polyglycolides, polycaprolactones, polyurethanes, polyesteramides, polyorthoesters, polydioxanones, polyacetals, polyketals, polycarbonates, polyorthocarbonates, polyphosphazenes, polyhydroxybutyrates, polyhydroxyvalerates, polyalkylene oxalates, polyalkylene succinates, poly(malic acid), poly(amino acids), polyvinylpyrrolidone, polyvinyl alcohol (PVA), polyalkylene glycols (PAG) such as polyethylene glycol, polyalkylcarbonate, chitin, chitosan, starch, fibrin, polyhydroxyacids such as polylactic acid and polyglycolic acid, poly(lactide-co-glycolide) (PLGA), poly(1-lactide-co-trimethylene carbonate), poly(d,1-lactide-co-trimethylene carbonate), poly(d,l-lactide), poly(d,1-lactide-co-glycolide), polyglycolide, polyhydroxycellulose, poly(butic acid), poly(valeric acid), proteins and polysaccharides such as collagen, hyaluronic acid, albumin, gelatin, cellulose, dextrans, fibrinogen, and blends and copolymers thereof In one embodiment, the biodegradable polymer is biocompatible, where a biocompatible polymer is a polymeric material that is compatible with living tissue or a living system, and is sufficiently non-toxic or non-injurious and causes minimal (if any) immunological reaction or rejection.

In one embodiment, the biodegradable polymer is selected from poly-1-lactide (PLLA), poly(lactide-co-glycolide) (PLGA), poly(1-lactide-co-trimethylene carbonate), poly(d,l-lactide-co-trimethylene carbonate), polyvinyl alcohol (PVA), polyalkylene glycols (PAG) such as polyethylene glycol (PEG), albumin, fibrin, gelatin, starch, cellulose, dextrans, collagen, hyaluronic acid, polysaccharides, fibrinogen, poly (D,L lactide), poly (D,L-lactide-co-glycolide), poly(glycolide), poly(hydroxybutyrate), poly(alkylcarbonate), poly(orthoesters), polycaprolactone, poly(ethylene terephthalate), poly(butyric acid), poly(valeric acid), polyanhydrides and blends and copolymers thereof, poly(hydroxyvalerate), poly(hydroxybutyrate, poly(hydroxybutyrate-co-valerate), polydioxanone, polyorthoesters, polyanhydrides, poly(glycolic acid-co-trimethylene carbonate), polyphosphoesters, polyphosphoester urethanes, polyamino acids, cyanoacrylates, poly(trimethylene carbonates), poly(iminocarbonate), copoly(ether-ester) (e.g., PEO/PLA), polyalkylene oxalates, polyphosphazenes, polypeptides, and proteins.

In one embodiment, the biodegradable polymer is chosen from PLLA, i.e., poly(1-lactide), the racemic version of PLA, i.e., poly(d,1-lactide), PLGA, i.e., poly (1- lactide-co-glycolide), the racemic version of PLGA, i.e., poly(d,l-lactide-co-glycolide), PLLC, i.e., poly(1-lactide-co-caprolactone and/or PDLLC, i.e., poly(d,l-lactide-co-caprolactone), poly(1-lactide-co-trimethylene carbonate) and poly(d,l-lactide-co-trimethylene carbonate).

In one embodiment, the device further comprises an additional protective coating containing no therapeutic agent. In one embodiment, the protective coating protects heparinized biodegradable polymers for controlled release from a variety of negative influences before and/or during implantation of the device. These influences can include exposure to air and/or light which may degrade they active agents and/or polymers, e.g., by oxidation, as well as to prevent the release of active ingredients during implantation of the device, before the devices reaches the site of implantation and already comes into direct contact with body fluids. The protective coating can be biocompatible and/or biodegradable, e.g., a soluble polymer under biological conditions. The composition and thickness of the protective coating can be chosen such that the coating will have sufficiently dissolved in a period between 30 minutes and several hours, in order not to unnecessarily delay the controlled release of the active agents. Exemplary suitable protective coatings can comprise any of the biodegradable polymers disclosed herein. In one embodiment, the protective coating is polyvinylpyrrolidone.

In one embodiment, the device comprises an inner coating that contacts the device and serves as a substrate for a coating comprising the biodegradable polymer. The inner coating can comprise a biodegradable polymer, as disclosed herein, or a nonbiodegradable polymer. Exemplary nonbiodegradable polymers include those polymers typically used as coatings for implantable medical devices, such as polyurethanes, polyacrylate esters, polyacrylic acid, polyvinyl acetate, and silicones. Other suitable nonbiodegradable polymers include styrene-isobutylene-styrene block copolymers such as styrene-isobutylene-styrene tert-block copolymers (SIBS); polyvinylpyrrolidone including cross-linked polyvinylpyrrolidone; polyvinyl alcohols, copolymers of vinyl monomers such as EVA; polyvinyl ethers; polyvinyl aromatics; polyethylene oxides; polyesters including polyethylene terephthalate; polyamides; polyacrylamides; polyethers including polyether sulfone; polyalkylenes including polypropylene, polyethylene and high molecular weight polyethylene; polycarbonates, siloxane polymers; cellulosic polymers such as cellulose acetate; polymer dispersions such as polyurethane dispersions (BAYHDROL^{®}); squalene emulsions; and mixtures and copolymers of any of the foregoing. In one embodiment, the nonbiodegradable polymer is selected from poly(n-butyl methacrylate)/poly(ethene vinyl acetate), polyacrylate, poly(lactide-co-E-caprolactone), phosphorylcholine, PTFE, paralyene C, polyethylene-co-vinyl acetate, poly n-butylmethacrylate, poly(styrene-b-isobutylene-b-styrene) (a tri-block copolymer of styrene and isobutylene subunits built on 1,3-di(2-methoxy-2-propyl)-5-tert-butylbenzene, Transelute^{™}).

In other embodiments, the inner coating can be a ceramic, such as those ceramics known in the art to be biocompatible, e.g., hydroxyapatite, titanium oxide, and silicon carbide. Exemplary treatments/coatings of a surface with a ceramic material that improves the performance of subsequently deposited polymer layer is disclosed in WO 2006/024125, the disclosure of which is incorporated herein by reference. Alternatively, the inner coating can be an inorganic coating, such as metals (e.g. gold), or carbon.

One of ordinary skill in the art can select suitable biodegradable or nonbiodegradable polymers, as these are well known in the art. Alternatively, screening methods can be used to select a biodegradable or nonbiodegradable polymer. For example, a biodegradable polymer can be subjected to suitable physiological conditions and monitored to assess whether they decompose, degenerate, degrade, depolymerize, or undergo any other mechanism that reduces the molecular weight of the polymer within an appropriate period of time, e.g., hours, days, depending on the particular application desired.

The devices of the invention may be coated partially or wholly with the above defined compositions in any manner known in the art, e.g., dipping, spraying, rolling, brushing, electrostatic plating or spinning, vapor deposition (e.g., physical or chemical), air spraying including atomized spray coating, and spray coating using an ultrasonic nozzle. The compositions can be applied by these methods either as a solid (e.g., film or particles), a suspension, a solution, or as a vapor. Alternatively, the device can be coated with a first substance (such as a hydrogel) that is capable of absorbing the composition. In another embodiment, the device can be constructed from a material comprising a polymer/drug composition.

In one embodiment, the heparinized polymer coating is combined with a therapeutically effective amount of the pharmaceutically active agent in an appropriate solvent, e.g., dichloromethane, to allow the agent to be evenly dispersed throughout the heparinized polymer matrix. "Therapeutically effective amount," as used herein which refers to that amount of agent that results in prevention or amelioration of symptoms in a patient or a desired biological outcome, e.g., improved clinical signs, delayed onset of disease, reduced/elevated levels of lymphocytes and/or antibodies, etc.

In one embodiment, the surface of the device is coated with at least one layer of heparinized polymer containing the selected drug in a preselected concentration. One or more additional layers of the heparinized polymer may also be coated on the device surface, each layer having a preselected concentration, e.g., a therapeutically effective amount of one or more of pharmaceutically active agents. Alternatively, one or more layers of non-heparinized polymer may be used, optionally in conjunction with heparinized polymer layers.

In one embodiment, the surface of the device (e.g., a bare metal surface of a stent) can be directly treated with the biodegradable coatings disclosed herein. In another embodiment, the bare metal surface of a device can be subjected to a pre-treatment for better polymer adhesion or compatibility such as roughening, sandblasting, oxidizing (e.g., with oxidizing acids), sputtering, plasma-deposition, physical vapor deposition, chemical vapor deposition, ionization, heating, photochemical activation, sintering, etching or priming with selected acids, organic solvents or other chemical substances designed/selected to render the stent surface more biocompatible, hydrophilic or hydrophobic as desired.

In one embodiment, the coating comprises heparin sodium coupled with a lactide based polymer such as poly(1-lactide) (PLLA) in combination with selected antiproliferative drugs.

Heparin can be covalently bound via a reaction protocol as, for example, described in Jee et al. For example, for polymers such as PLA (e.g., the d,l moiety) and PLGA (e.g., the 1 and d,1-co-glycolide moieties) as well as other related lactide, co-glycolide and co-caprolactone (PLLC and PDLLC) and co-trimethylene carbonate biodegradable polymers as described herein, heparin can be covalently bound to the terminal hydroxyl groups of the PLA, PLLA, PLGA or the like lactide based biodegradable polymers. In one embodiment, covalent attachment of heparin to a lactide based biodegradable polymer can be achieved as follows:

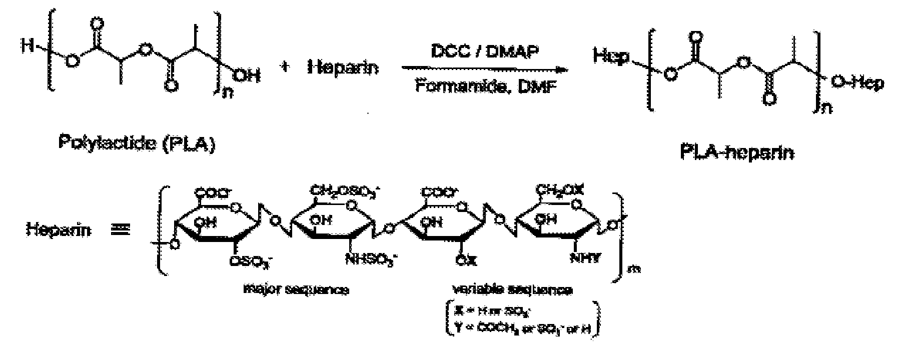

### Pharmaceutically Active Agents

In one embodiment, at least one pharmaceutically active agent other than heparin is dispersed within the at least one polymer. By "dispersed," the agent can be adhered to the polymer, entrapped by the polymer, or bound covalently or ionically to the polymer. The agent can be present in the surface and/or the bulk of the polymer layer.

In embodiments where the coating comprises a polymer having a COOH or other electrophilic group available for reaction, a pharmaceutically active agent that has a nucleophlic group available for reaction such as hydroxyl or amine can be covalently linked to the electrophilic group containing polymer by a reaction protocol that activates the electrophilic group such that the nucleophilic group of the pharmaceutically active agent covalently bonds to the COOH or other electrophilic group of the polymer.

A typical polymer for use in covalent bonding to an active agent is a biodegradable polymer having terminal COOH groups such as lactide based polymers such as PLLA, PLGA, PLA or the like. Other biodegradable polymers with a single terminal COOH include poly-glycine, poly-D,L alanine, poly L-alanine, poly-L-asparagine and poly-amino acids generally. Other suitable biodegradable polymers with COOH side chains include poly-(alpha, beta)-D,L-aspartic acid, poly-L aspartic acid, poly-(D,L)-glutamic acid, poly-L-glutamic acid and other similar polymers.

The polymer is first dissolved in a suitable solvent such as dichloromethane in the case of PLLA. One equivalent of an activating substance such as dicyclohexylcarbodiimide (DCC) is added to the PLLA solution. In this example, a solution of one equivalent of the pharmaceutically active agent, LY303511 which has an amine function dissolved in DMF is then immediately added to the dichloromethane/polymer solution. The reaction mixture is stirred at 40-50° C for 4-18 hours until the reaction is complete as indicated by thin layer chromatography. The pharmaceutically active agent is thus covalently bound to the polymer.

In another specific example, the pharmaceutically active agent Genistein and DMAP are dissolved in a suitable solvent such as DMF (dimethylformamide) and DCC is then added (i.e. molar amounts of DCC and Genistein are equivalent). PLLA is dissolved in dichloromethane. The solution of PLLA is then added dropwise to the Genistein solution, heated to about 50 °C and stirred for about 18 hours. The reaction product of Genistein and PLLA is then precipitated from the reaction solution by addition of methanol. The precipitate is purified by dissolution in chloroform, precipitated again out of solution with methanol and washed and dried for later use in a coating process that deposits the genisteinized PLLA on the surface of a stent, balloon component of a balloon catheter or other medical device.

The same or similar covalent bonding process may be carried out with other pharmaceutically active agents having a nucleophilic group available for reaction with a complementary electrophilic group on the polymer coating material including those active agents identified/disclosed herein, such as rapamycin and its analogs, paclitaxel and its analogs, flavonoids and benzopyran-4-one compounds described or identified herein. Carbonyl activating agents other than DCC may also be employed such as N-(3-dimethylaminopropyl)-N'-ethyl-carbodiimide hydrochloride and carbonyldiimidazole.

In one embodiment, the at least one pharmaceutically active agent is chosen from antithrombotics, anticoagulants, antiplatelet agents, thrombolytics, antiproliferatives, anti-inflammatories, antimitotic, antimicrobial, agents that inhibit restenosis, smooth muscle cell inhibitors, antibiotics, fibrinolytic, immunosuppressive, and anti-antigenic agents.

Exemplary pharmaceutically active agents include cell cycle inhibitors in general, apoptosis-inducing agents, antiprofiferative/antimitotic agents including natural products such as vinca alkaloids (e.g., vinblastine, vincristine, and vinorelbine), paclitaxel, colchicine, epidipodophyllotoxins (e.g., etoposide, teniposide), antibiotics (e.g., dactinomycin, actinomycin D, daunorubicin, doxorubicin, idarubicin, penicillins, cephalosporins, and quinolones), anthracyclines, mitoxantrone, bleomycins, plicamycin (mithramycin), mitomycin, enzymes (e.g., L-asparaginase, which systemically metabolizes L-asparagine and deprives cells that do not have the capacity to synthesize their own asparagine); antiplatelet agents such as G(GP) II_{b}/IIIₐ inhibitors, GP-IIa inhibitors and vitronectin receptor antagonists; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, streptozocin), triazenes--dacarbazine (DTIC); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine (cladribine)); platinum coordination complexes (cisplatin, carboplatin), procarbazine, hydroxyurea, mitotane, aminoglutethimide; hormones (e.g., estrogen); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); anti-inflammatory: such as adrenocortical steroids (cortisol, cortisone, fluorocortisone, prednisone, prednisolone, 6α-methylprednisolone, triamcinolone, betamethasone, and dexamethasone), non-steroidal agents (salicylic acid derivatives e.g., aspirin; para-aminophenol derivatives e.g., acetominophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); antigenic agents: vascular endothelial growth factor (VEGF), fibroblast growth factor (FGF); angiotensin receptor blockers; nitric oxide donors; anti-sense oligionucleotides and combinations thereof; cell cycle inhibitors, mTOR inhibitors, and growth factor receptor signal transduction kinase inhibitors; retinoid; cyclin/CDK inhibitors; HMG co-enzyme reductase inhibitors (statins); and protease inhibitors (matrix protease inhibitors).

In one embodiment, the pharmaceutically active agent is an agent for preventing or reducing restenosis, e.g., for preventing or reducing restenosis subsequent to or associated with angioplasty. In one embodiment, the therapeutic agent exhibits synergy with a flavonoid as defined herein in preventing or reducing restenosis as well as in preventing or reducing secondary complications after angioplasty, including, e.g., acute, subacute and chronic secondary complications associated with angioplasty such as thrombus, inflammation, and responses of the immune system. Suitable second or further pharmaceutically active agents that exhibit synergy with the flavonoid as defined above include agents that are useful for treating restenosis and include known anti-inflammatory, anti-thrombogenic, anti-antigenic, matrix protease inhibitory, anti-migratory, anti-proliferative (e.g., a tubulin-binding anti-proliferative agent), cytostatic, and/or cytotoxic agents. Exemplary agents are those that are currently being used or considered as stent coating materials to combat restenosis, which include paclitaxel, derivatives of paclitaxel, sirolimus, and derivatives of sirolimus.

Analogs or derivatives of paclitaxel include docetaxel, BMS-184476, BMS 275183, BAY 59-8862, orataxel, taxumairol, taxinine M, taxacin, various baccatines and others described by Ya-Ching Shen et al., 2000, J. Chin. Chem. Soc., 47: 1125-30; Plummer et al.,2002, Clin Cancer Res. 8:2788-97; Agarwal et al., 2003, Curr. Oncol. Rep. 5: 89-98; and Jordan and Wilson, 2004, Nature Rev. Cancer 4: 253-65.

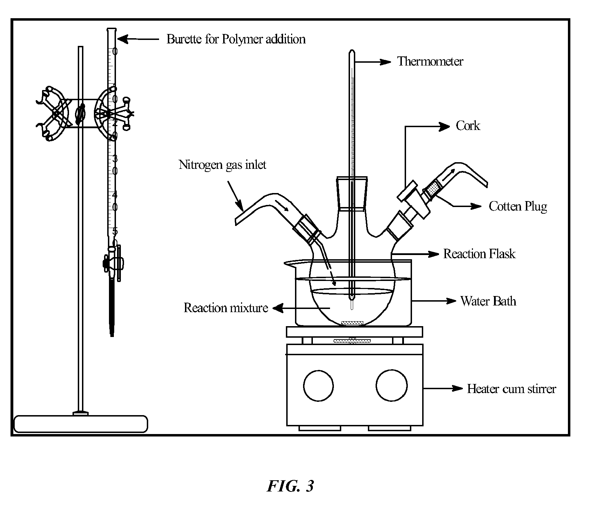

In one embodiment, the pharmaceutically active agent is an anti-proliferative agent. Sirolimus is one drug that has been shown to work well as a potent anti-proliferative drug that prevents excess intimal growth. Sirolimus and its active analogs/derivatives only becomes active after forming a complex with intracellular binding proteins known as immunophilins. Sirolimus binds to a family of immunophilins called the FK-binding proteins. Sirolimus binds to FK506-binding protein, FKBP, which is the same molecule that is bound by FK506. The complex that is formed between Sirolimus and FKBP binds to the mammalian target of Rapamycin (mTOR). The sirolimus-FKBP-mTOR complex inhibits biochemical pathways that are required for cell progression late into the G1 phase or entry into the S phase of the cell cycle (G1/S cell cycle arrest) whereby cell proliferation and the cell structure remains stable and viable. This is graphically represented in FIG. 3.

Analogs of sirolimus (rapamycin) include C-7 Rapalog, AP 22594, 28-epirapamycin, 24,30-tetrahydro-rapamycin, AP 23573, trans-3-aza-bicyclo[3.1.0] hexane-2-carboxylic acid Rapamycin, ABT-578, SDZ RAD, CCI-779, AP 20840, AP 23464.

Other exemplary pharmaceutically active agents include flavonoids. Flavonoids are polyphenolic substances based on a flavan nucleus, comprising 15 carbon atoms, arranged in three rings as C₆--C₃--C₆ with a general structure according to Formula I:

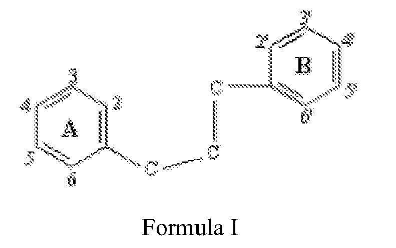

The chemical structure of flavonoids are based on a C₁₅ skeleton with a chromane ring bearing a second aromatic ring B in position 2, 3 or 4 (Formula II). In a few cases, the six-membered heterocyclic ring C occurs in an isomeric open form or is replaced by a five- membered ring.

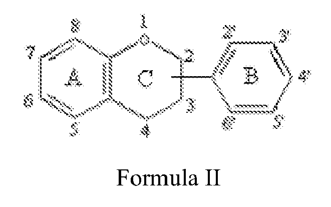

Flavonoids are biosynthetically derived from acetate and shikimate such that the A ring has a characteristic hydroxylation pattern at the 5 and 7 position. The B ring is usually 4', 3'4', or 3'4'5'-hydroxylated. Flavonoids have generally been classified into 12 different subclasses by the state of oxidation and the substitution pattern at the C2-C3 unit. There are a number of chemical variations of the flavonoids, such as, the state of oxidation of the bond between the C2-C3 position and the degree of hydroxylation, methoxylation or glycosylation (or other substituents) in the A, B and C rings and the presence or absence of a carbonyl at position 4. Flavonoids for use in the present invention include, but are not limited to, members of the following subclasses: chalcone, dihydrochalcone, flavanone, flavonol, dihydroflavonol, flavone (found in citrus fruits), flavanol, isoflavone, neoflavone, aurone, anthocyanidin (found in cherries, strawberries, grapes and colored fruits), proanthocyanidin (flavan-3,4-diol) and isoflavane. Thus far, more than 10,000 flavonoids have been identified from natural sources. Berhow (1998) pp. 67-84 in Flavonoids in the Living System, ed. Manthey et al., Plenum Press, NY.

In one embodiment, unless otherwise specified, "substituted" or "substituent" refers to substitutions with at least one of the following groups: alkyl, cycloalkyl, alkoxy, amino, amido, aryl, carboxy, cyano, cycloalkyl, halogen, hydroxy, nitro. In one embodiment, "alkyl" refers to a C₁-C₂₀ alkyl, such as a C₁-C₁₂ alkyl, or a C₁-C₆ alkyl. In one embodiment, "heteroalkyl" refers to alkyl groups substituted with at least one of O, N, S, or halogen. In one embodiment "aryl" comprises mono-, bi-, or multi- carbon-based, aromatic rings, e.g., phenyl and naphthyl. In one embodiment, "heteroaryl" refers to an aryl as defined herein, wherein a ring carbon is replaced with at least, one of O, N, or S.

Flavonoids have a number of activities that are useful in the context of the present invention. These activities include e.g. anti-platelet aggregation, anti-thrombotic, anti-inflammatory, anti-atherogenic, anti-oxidant, inhibition of angiogenesis, inhibition of lipid oxidation and peroxidation, lipid-lowering and inhibition of cell cycle. In one embodiment, a composition of the present invention at least comprises a flavonoid with anti-platelet aggregation activity and/or anti-thrombotic activities. These activities may be assayed by methods know to the skilled person per se (see e.g. E. M. Van Cott, M.D., and M. Laposata, M.D., Ph.D., PCoagulation" In: Jacobs DS et al, ed. "The Laboratory Test Handbook," 5th Edition. Lexi-Comp, Cleveland, 2001; 327-358). A composition of the invention may however comprises more than one flavonoid. For example, at least one flavonoid comprises anti-platelet aggregation activity and/or anti-thrombotic activities and the other flavonoid(s) comprise other useful activities as indicated above.

An exemplary flavonoid for use in the compositions of the present invention is a flavonoid that mediates the above anti-platelet aggregation, anti-thrombotic and anti-inflammatory activities through their ability to inhibit DNA topoisomerase II, protein tyrosine kinases, and/or nitric oxide synthase and/or modulation of the activity of NF-kappaB. These activities may be assayed by methods known to the skilled person per se (see, e.g., Andrea et al., 1991, Mol. Pharmacol. 40:495-501; the HitHunter EFC-TK assay from DiscoverX, Fremont, CA; Webb and Ebeler, 2004, Biochem. J. 384: 527-41; Akiyama et al., 1987, J. Biol. Chem., Vol. 262, 5592-95).

Further properties of the flavonoids that are relevant in the context of the present invention include: inhibition of cell cycle, inhibition of smooth muscle cell proliferation and/or migration. A flavonoid, in one embodiment, is capable of exerting the above activities when used singly. However, the above properties of the flavonoid may be further enhance by exploiting the synergy between the flavonoid and further therapeutic agents (as listed below herein), e.g., paclitaxel, sirolimus and/or rapamycin.

A flavonoid for use in the compositions of the present invention may be selected from narigenin, naringin, eriodictyol, hesperetin, hesperidin (esperidine), kampferol, quercetin, rutin, cyanidol, meciadonol, catechin, epi-gallocatechin-gallate, taxifolin (dihydroquercetin), genistein, genistin, daidzein, biochanin, glycitein, chrysin, diosmin, luetolin, apigenin, tangeritin and nobiletin. In one embodiment, a flavonoid for use in the compositions of the present invention is a flavanone, a flavonol, or an isoflavone. In another embodiment, the flavonoid is selected from genistein, quercetin, rutin, narigenin and naringin. Alternatively, a mixture of flavonoids extracted from plant-material may be used in the composition of the invention such as e.g. extracts from grapes *(Vitis vinifera),* e.g., grape seed or grape skin (see e.g. Shanmuganayagam et al., 2002, J. Nutr. 132:3592-98). Furthermore, derivatives of the above flavonoids may be used in the compositions of the invention. By "derivative" is meant a compound derived from and thus non-identical to another compound. As used herein, a derivative shares at least one function with the compound from which it is derived, but differs from that compound structurally. Derivatives of flavonoids include without limitation those that differ from flavonoids due to modifications (including without limitation substitutions, additions and deletions) in a ring structure or side chain. Derivatives of flavonoids include those compounds which differ from flavonoids in structure. These structural differences can be, as non-limiting examples, by addition, substitution or re-arrangement of hydroxyl, alkyl or other group. As a non-limiting example, a flavonoids derivative can have additional alkyl groups attached. In addition, flavonoids derivatives include compounds which have been conjugated to another chemical moiety, such as a sugar or other carbohydrate. Derivatives also include salts of flavonoids.

In one embodiment, a flavonoid for use in the compositions of the present invention is genistein or an analog of genistein. Genistein is the aglycone (aglucon) of genistin. The isoflavone is found naturally as the glycoside genistin and as the glycosides 6"-O-malonylgenistin and 6"-O-acetylgenistin. Genistein and its glycosides are mainly found in legumes, such as soybeans and chickpeas. Genistein is a solid substance that is practically insoluble in water. Its molecular formula is C₁₅H₁₀O₅, and its molecular weight is 270.24 daltons. Genistein is also known as 5, 7-dihydroxy-3- (4-hydroxyphenyl)-4H-1-benzopyran-4-one, and 4', 5, 7-trihydroxyisoflavone. Genistin, which is the 7-beta glucoside of genistein, has greater water solubility than genistein. Genistein has the following structural formula:

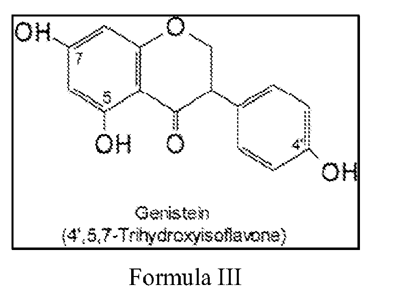

Genistein has been found to have a number of antioxidant activities. It is a scavenger of reactive oxygen species and inhibits lipid peroxidation. It also inhibits superoxide anion generation by the enzyme xanthine oxidase. In addition, genistein, in animal experiments, has been found to increase the activities of the antioxidant enzymes superoxide dismutase, glutathione peroxidase, catalase and glutathione reductase. Genistein's activities include upregulation of apoptosis, inhibition of angiogenesis, inhibition of platelet aggregation, inhibition of DNA topoisomerase II and inhibition of protein tyrosine kinases. Genistein has been reported to have anti-carcinogenic activity, anti-atherogenic activity, lipid-lowering activity, and it may help protect against osteoporosis. A genistein or an analog thereof for use in the present invention can be an inhibitor of tyrosine kinases (as may be assayed as indicated above). Alternatively, other tyrosine kinase inhibitors may be used instead of genistein in the context of the invention, including e.g. erbstatin, herbamycin A, lavendustine-c and hydroxycinnamates. A genistein or an analog thereof for use in the present invention can be an DNA topoisomerase II inhibitor (as may be assayed as indicated above). A genistein or an analog thereof for use in the present invention can be an inhibitor of platelet aggregation and therefore, an inhibitor of thrombus formation (as may be assayed as indicated above). Further properties of genistein or its analogs that are relevant in the context of the present invention include: inhibition of cell cycle, inhibition of smooth muscle cell proliferation and/or migration.

Genistein and/or its analogs may be capable of exerting the above activities when used singly. However, the above properties of genistein and/or its analogs may be further enhance by exploiting the synergy between genistein and/or its analogs and further therapeutic agents (as listed herein below), e.g., paclitaxel, sirolimus and/or rapamycin. Analogs of genistein include genistin and daidzein.

Another exemplary flavonoid for use in the compositions of the present invention is quercetin or an analog of quercetin. Quercetin is typically found in plants as glycone or carbohydrate conjugates. Quercetin itself is an aglycone or aglucon. That is, quercetin does not possess a carbohydrate moiety in its structure. Analogs of quercetin include its glycone conjugates include rutin and thujin. Rutin is also known as quercetin-3-rutinoside. Thujin is also known as quercitrin, quercetin-3-L-rhamnoside, and 3-rhamnosylquercetin. Onions contain conjugates of quercetin and the carbohydrate isorhamnetin, including quercetin-3,4'-di-O-beta glucoside, isorhamnetin-4'-0-beta-glucoside and quercetin-4'-0-beta-glucoside. Quercetin itself is practically insoluble in water. The quercetin carbohydrate conjugates have much greater water solubility then quercetin.

Quercetin is known chemically as 2-(3, 4-dihydroxyphenyl)-3,5,7-trihydroxy-4H-1-benzopyran-4-one and 3,3',4'5,7-pentahydroxy flavone. It is also known as meletin and sophretin and is represented by the following structural formula:

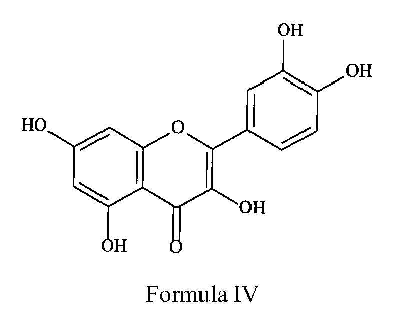

Quercetin is a phenolic antioxidant and has been shown to inhibit lipid peroxidation. *In vitro* and animal studies have shown that quercetin inhibits degranulation of mast cells, basophils and neutrophils. Such activity account, in part, for quercetin's anti-inflammatory and immunomodulating activities. Other *in vitro* and animal studies show that quercetin inhibits tyrosine kinase and nitric oxide synthase and that it modulates the activity of the inflammatory mediator, NF-kappaB. Further activities of quercetin include anti-viral and anti-cancer activity. Quercetin is further known to inhibit aldose reductase. A quercetin or an analog thereof for use in the present invention can be an inhibitor of tyrosine kinases (as may be assayed as indicated above). Alternatively, other tyrosine kinase inhibitors (indicated above) may be used instead of quercetin in the context of the invention (as may be assayed as indicated above). A quercetin or an analog thereof for use in the present invention can be an nitric oxide synthase inhibitor (as may be assayed as indicated above). A quercetin or an analog thereof for use in the present invention can be an inhibitor of platelet aggregation and therefore, an inhibitor of thrombus formation (as may be assayed as indicated above).

Further properties of quercetin or its analogs that are relevant in the context of the present invention include: inhibition of cell cycle, inhibition of smooth muscle cell proliferation and/or migration. Suitable analogs/derivatives of quercetin include its glycone conjugates rutin and thujin.

Quercetin and/or its analogs may be capable of exerting the above activities when used singly. However, the above properties of quercetin and/or its analogs may be further enhance by exploiting the synergy between quercetin and/or its analogs and further therapeutic agents (as disclosed herein), such as paclitaxel, and/or sirolimus (rapamycin).

In one embodiment, the flavonoid is selected from genistein, quercetin, rutin, narigenin, naringin, and derivatives thereof.

In one embodiment, the agent is selected from 2-(4-piperazinyl)-substituted 4H-1 benzopyran-**4**-one compounds having the structure of Formula A: 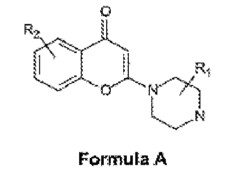 wherein the presence of each of R1 and R2 is optional and R1 and R2 are each independently selected from alkyl, substituted alkyl, heteroalkyl, cycloalkyl, substituted cycloalkyl, heterocycloalkyl, substituted heterocycloalkyl, aryl, substituted aryl, heteroaryl, substituted heteroaryl, alkoxy, halogen, hydroxy, and amino.

In another embodiment, the agent is selected from compounds having the structure of Formula B: 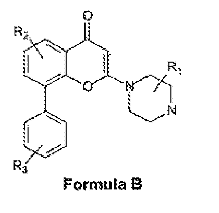 wherein the presence of each of R1 and R2 and R3 is optional and R1 and R2 and R3 are each independently selected from alkyl, aryl, alkoxy, halogen, hydroxy or amino. If R1 or R2 or R3 are present, there may be one or more R1 or R2 or R3 substituents on each respective structure.

An example of the above Formula A and B molecules include a molecule designated LY303511:

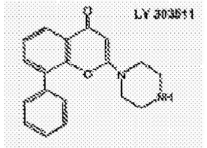

Another example of a benzopyran-4-one compound is designated LY294002.

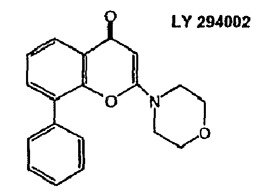

The dihydrochloride salt of 2-(4-piperazinyl)-8-phenyl-4H-1-benzopyran-4-one is commercially available from Sigma-Aldrich Corporation under the designation "LY303511." The 2-(4-piperazinyl)-8-phenyl-4H-1-benzopyran-4-one compounds described herein may be synthesized based on the procedures described in Vlahos et al., J. Biol. Chem. 269: 5241-5248,1994. Other salt forms of 2- (4-piperazinyl)-8-phenyl-4H-1-benzopyran-4-one are readily synthesizable following known techniques such as those described in Handbook of Pharmaceutical Salts, Properties, Selection and Use, Wiley VCH (2002).

In one embodiment, the pharmaceutically active agents are chosen from paclitaxel, rapamycin, genistein, sirolimus, tacrolimus and everolimus, and derivatives and analogs thereof.

In one embodiment, the combination of heparinized polymer and pharmaceutically active agent comprise a combination of pharmaceutically active agents. If more than one pharmaceutically active agent is used, they can be present in combination in the same layer, or in separate heparinized polymer layers. Exemplary combinations include genistein plus sirolimus separately or in combination in one or more coatings and genistein and LY303511 or in combination in one or more coatings.

### Dosages

On-stent dosages of the at least one pharmaceutically active agent may be determined by means known in the art. Typically, the dosage is dependent upon the particular drug employed and medical condition being treated to achieve a therapeutic result. In one embodiment, the amount of drug represents about 0.001 percent to about seventy percent of the total coating weight, or about 0.01 percent to about sixty percent of the total coating weight. In one embodiment, the weight percent of the therapeutic agents in the carrier or polymer coating is 1% to 50%, 2% to 45, 5% to 40, or 10 to 35%. In another embodiment, it is possible that the drug may represent as little as 0.0001 percent to the total coating weight. In another embodiment, the amount of selected drugs loaded onto a 16mm long stent range from about 30 to about 105 micrograms per coating layer.

In one embodiment, the dosage or concentration of, e.g., paclitaxel based on surface area on a typical coronary stent can range from about 0.1 to about 5 µg/mm², or more than about 0.7 µg/mm² (at lower dosage restenosis rates are higher), or less than about 3.0 µg/mm² (higher will be cytotoxic), or ranging from 1.0 and 1.8 µg/mm², and or about 1.4 µg/mm². Typically, the amount of paclitaxel will increase linearly with the length of the stent. In one embodiment, for a typical series of coronary stent varying in length from 8.00 to 39.00 mm, the total paclitaxel content will vary from 50 µg to 250 µg. Suitable dosaging for drug-eluting stents is further described in US 6,908,622, the disclosure of which is incorporated herein by reference.

The dosage or concentration of e.g. sirolimus based on surface area on a typical coronary stent may be is 0.1 and 5 µg/mm². In another embodiment, the dosage is more than about 0.7 µg/mm² (at lower dosage restenosis rates are higher) and less than about 3.0 µg/mm² (higher will be cytotoxic), such as ranging from 1.0 and 1.8 µg/mm², e.g., about 1.4 µg/mm². Typically, the amount of sirolimus will increase linearly with the length of the stent. For example, for a typical series of coronary stent varying in length from 8.00 to 39.00 mm, the total sirolimus content will vary from 50 µg to 250 µg.

The dosage or concentration of a flavonoid or derivative thereof based on surface area on a stent (e.g. a typical coronal stent) may be is 0.1 and 40 µg/mm². In one embodiment, the dosage of a flavonoid or derivative thereof based on surface area of a device of the invention is more than about 0.2., 0.5, 1.0, 2.0, 5.0 or 10 µg/mm². In another embodiment, the dosage of a flavonoid or derivative thereof based on surface area of a device of the invention is less than about 30.0, 20.0, 15.0, 10.0, 5.0, 3.0 or 2.0 µg/mm². Generally, the amount of the flavonoid or derivative thereof will increase linearly with the length of the stent. For example, for a typical series of coronary stent varying in length from 8.00 to 39.00 mm, the total flavonoid (or derivative thereof) content will vary from 28 µg to 3500 µg.

### Devices

In one embodiment, the device treats narrowing or obstruction of a body passageway in a subject in need thereof. In another embodiment, the method comprises inserting the device into the passageway, the device comprising a generally tubular structure, the surface of the structure being coated with a composition disclosed herein, such that the passageway is expanded. In the method, the body passageway may be selected from arteries, veins, lacrimal ducts, trachea, bronchi, bronchiole, nasal passages, sinuses, eustachian tubes, the external auditory canal, oral cavities, the esophagus, the stomach, the duodenum, the small intestine, the large intestine, biliary tracts, the ureter, the bladder, the urethra, the fallopian tubes, uterus, vagina, the vasdeferens, and the ventricular system.

Exemplary devices include sutures, staples, anastomosis devices, vertebral disks, bone pins, suture anchors, hemostatic barriers, clamps, screws, plates, clips, vascular implants, urological implants, tissue adhesives and sealants, tissue scaffolds, bone substitutes, intraluminal devices, and vascular supports. For example, the device can be a cardiovascular device, such as venous catheters, venous ports, tunneled venous catheters, chronic infusion lines or ports, including hepatic artery infusion catheters, pacemakers and pace maker leads, and implantable defibrillators. Alternatively, the device can be a neurologic/neurosurgical device such as ventricular peritoneal shunts, ventricular atrial shunts, nerve stimulator devices, dural patches and implants to prevent epidural fibrosis post-laminectomy, and devices for continuous subarachnoid infusions. The device can be a gastrointestinal device, such as chronic indwelling catheters, feeding tubes, portosystemic shunts, shunts for ascites, peritoneal implants for drug delivery, peritoneal dialysis catheters, and suspensions or solid implants to prevent surgical adhesions. In another example, the device can be a genitourinary device, such as uterine implants, including intrauterine devices (IUDs) and devices to prevent endometrial hyperplasia, fallopian tubal implants, including reversible sterilization devices, fallopian tubal stents, artificial sphincters and periurethral implants for incontinence, ureteric stents, chronic indwelling catheters, bladder augmentations, or wraps or splints for vasovasostomy, central venous catheters.

Other exemplary devices include prosthetic heart valves, vascular grafts ophthalmologic implants (e.g., multino implants and other implants for neovascular glaucoma, drug eluting contact lenses for pterygiums, splints for failed dacrocystalrhinostomy, drug eluting contact lenses for corneal neovascularity, implants for diabetic retinopathy, drug eluting contact lenses for high risk corneal transplants), otolaryngology devices (e.g., ossicular implants, Eustachian tube splints or stents for glue ear or chronic otitis as an alternative to transtempanic drains), plastic surgery implants (e.g., breast implants or chin implants), and catheter cuffs and orthopedic implants (e.g., cemented orthopedic prostheses).

Another exemplary device according to the invention is a stent, such as a stent comprising a generally tubular structure. A stent is commonly used as a tubular structure disposed inside the lumen of a duct to relieve an obstruction. Commonly, stents are inserted into the lumen in a non-expanded form and are then expanded autonomously, or with the aid of a second device in situ. A typical method of expansion occurs through the use of a catheter-mounted angioplasty balloon which is inflated within the stenosed vessel or body passageway in order to shear and disrupt the obstructions associated with the wall components of the vessel and to obtain an enlarged lumen.

An exemplary stent is a stent for treating narrowing or obstruction of a body passageway in a human or animal in need thereof. "Body passageway" as used herein refers to any of number of passageways, tubes, pipes, tracts, canals, sinuses or conduits which have an inner lumen and allow the flow of materials within the body. Representative examples of body passageways include arteries and veins, lacrimal ducts, the trachea, bronchi, bronchiole, nasal passages (including the sinuses) and other airways, eustachian tubes, the external auditory canal, oral cavities, the esophagus, the stomach, the duodenum, the small intestine, the large intestine, biliary tracts, the ureter, the bladder, the urethra, the fallopian tubes, uterus, vagina and other passageways of the female reproductive tract, the vasdeferens and other passageways of the male reproductive tract, and the ventricular system (cerebrospinal fluid) of the brain and the spinal cord. Exemplary devices of the invention are for these above-mentioned body passageways, such as stents, e.g., vascular stents. There is a multiplicity of different vascular stents known in the art that may be utilized following percutaneous transluminal coronary angioplasty.

Any number of stents may be utilized in accordance with the present invention and the invention is not limited to the specific stents that are described in exemplary embodiments of the present invention. The skilled artisan will recognize that any number of stents may be utilized in connection with the present invention. In addition, as stated above, other medical devices may be utilized, such as e.g., orthopedic implants.

### Methods of Treatment

In one embodiment, the implantable devices disclosed herein are implanted in a subject in need thereof to achieve a therapeutic effect, e.g., therapeutic treatment and/or prophylactic/preventative measures. Those in need of treatment may include individuals already having a particular medical disease as well as those at risk for the disease (e,g., those who are likely to ultimately acquire the disorder). A therapeutic method can also result in the prevention or amelioration of symptoms, or an otherwise desired biological outcome, and may be evaluated by improved clinical signs, delayed onset of disease, reduced/elevated levels of lymphocytes and/or antibodies.

In one embodiment, the method is used for treating at least one disease or condition associated with vascular injury or angioplasty. Angioplasty may be performed as part of "revascularization" treatment for "artherosclerosis," which as used herein means diseases in which plaque, made up of cholesterol, fats, calcium, and scar tissue, builds up in the wall of blood vessels, narrowing the lumen and interfering with blood flow. "Revascularization," as used herein means any treatment that re-establishes brisk blood flow through a narrowed artery, including bypass surgery, angioplasty, stenting, and other interventional procedures. Secondary complications following revascularization may include restenosis, neointima, neointimal hyperplasia and thrombosis. "Restenosis," as used herein is defined as the re-narrowing of an artery in the same location of a previous treatment; clinical restenosis is the manifestation of an ischemic event, usually in the form of recurrent angina. "Neointima," as used herein is defined as the scar tissue made up of cells and cell secretions that often forms as a result of vessel injury following angioplasty or stent placement as part of the natural healing process. "Neointimal hyperplasia," as used herein means excessive growth of smooth muscle cells from the inner lining of the artery. After angioplasty and/or stenting, excessive growth of these cells can narrow the artery again. "Thrombosis," as used herein means the formation of a blood clot within a blood vessel or the heart cavity itself and a "thrombus" is a blood clot.

Three pathophysiological phases can be distinguished subsequent to revascularization. Stage I, the thrombotic phase (days 0-3 after revascularization). This stage consists of rapid thrombus formation. The initial response to arterial injury is explosive activation, adhesion, aggregation, and platelet deposition. The platelet thrombus may frequently be large and can grow large enough to occlude the vessel, as occurs in myocardial infarction. Within 24 hours, fibrin-rich thrombus accumulates around the platelet site. Two morphologic features are prominent: 1) platelet/fibrin, and 2) fibrin/red cell thrombus. The platelets are densely clumped at the injury site, with the fibrin/ red cell thrombus attached to the platelet mass.

Stage II, the recruitment phase (days 3-8). The thrombus at arterial injury sites develops an endothelial cell layer. Shortly after the endothelial cells appear, an intense cellular infiltration occurs. The infiltration is principally monocytes that become macrophages as they leave the bloodstream and migrate into the subendothelial mural thrombus. Lymphocytes also are present, and both types of cells demarginate from the bloodstream. This infiltrate develops from the luminal side of the injured artery, and the cells migrate progressively deeper into the mural thrombus.

Stage III, the proliferative phase: (day 8 to final healing). Actin-positive cells colonize the residual thrombus from the lumen, forming a "cap" across the top of the mural thrombus in this final stage. The cells progressively proliferate toward the injured media, resorbing thrombus until it is completely gone and replaced by neointimal cells. At this time the healing is complete. In the pig this process requires 21-40 days, depending on residual thrombus thickness. Smooth muscle cell migration and proliferation into the degenerated thrombus increases neointimal volume, appearing greater than that of thrombus alone. The smooth muscle cells migrate from sites distant to the injury location, and the resorbing thrombus becomes a bioabsorbable "proliferation matrix" for neointimal cells to migrate and replicate. The thrombus is colonized at progressively deeper levels until neointimal healing is complete.

In one embodiment, the method of the invention can be used to treat these conditions subsequent to revascularization, such as those conditions subsequent to any of the three stages described above, e.g., activation, adhesion, aggregation, platelet deposition, thrombosis, platelet aggregation, proliferation, and neointima.

In one embodiment, the medicament is for the prevention or treatment of restenosis subsequent to angioplasty, such as the inhibition of neointimal hyperplasia subsequent to angioplasty.

In one embodiment, the methods of the invention are directed to the prevention of acute, subacute and chronic secondary complications associated with angioplasty. Such secondary complications subsequent to and/or associated with angioplasty are defined herein above and include, e.g., restenosis, neointima, neointimal hyperplasia, thrombosis and inflammation.

In one embodiment, the methods disclosed herein are directed to treating undesired cell proliferation, which is often a component of many disease processes. For example, undesired cell growth can lead to the formation of either benign or malignant tumors. Tumors include hematological tumors and solid tumors. Exemplary tumors are tumors of the skin, nervous system, lung, breast, reproductive organs, pancreas, lymphoid cells (including leukemias and lymphomas), blood or lymphatic vessels, and colon. Tumors include carcinomas, sarcomas, papillomas, adenomas, leukemias, lymphomas, melanomas, and adenocarcinomas.

Undesired cell growth can also be a component of restenosis, the recurrence of stenosis or artery stricture after corrective surgery. Restenosis occurs after coronary artery bypass (CAB),endarterectomy, heart transplantation, or after angioplasty, atherectomy, laser ablation or stenting. Restenosis is the result of injury to the blood vessel wall during the lumen opening procedure. In some patients, the injury initiates a repair response that is characterized by smooth muscle cell proliferation referred to as "hyperplasia" in the region traumatized by the angioplasty. This proliferation of smooth muscle cells re-narrows the lumen that was opened by the angioplasty within a few weeks to a few months, thereby necessitating a repeat angioplasty or other procedure to alleviate the restenosis.

The therapeutic compounds disclosed herein can be used to treat restenosis by administering the compound to the patient prior to, during and/or after coronary-or peripheral-artery angioplasty or atherectomy, coronary bypass graft or stent surgery, or peripheral vascular surgery (e.g., carotid or other peripheral vesselendarterectomy, vascular bypass, stent or prosthetic graft procedure). The benzopyran-4-ones may be delivered via luminal devices such as vascular stents or grafts. For example, a coated stent or graft as disclosed herein may be implanted at the vascular site of interest for controlled release of the pharmaceutically active agents over a desired time period.

In another embodiment, the method is directed to treating autoimmune diseases. An "autoimmune disease" is a disease in which the immune system produces an immune response (e. g., a B cell or a T cell response) against an antigen that is part of the normal host (i.e., an autoantigen), with consequent injury to tissues. An autoantigen may be derived from a host cell, or may be derived from a commensal organism such as themicro-organisms (known as commensal organisms) that normally colonize mucosal surfaces. In an immune response, T and or B cells proliferate in response to a stimulus viewed as "exogenous" by the immune system. Although generally, immune responses are beneficial, there are situations where a decreased immune response is desired. For example, in autoimmune disorders, the cells of the immune system incorrectly identify a self component as exogenous and proliferate in response to the self component.

Exemplary autoimmune diseases affecting mammals include rheumatoid arthritis, juvenile oligoarthritis, collagen-induced arthritis, adjuvant-induced arthritis, Sjogren's syndrome, multiple sclerosis, experimental autoimmuneencephalomyelitis, inflammatory bowel disease (e.g., Crohn's disease, ulcerative colitis), autoimmune gastric atrophy, pemphigusvulgaris, psoriasis, vitiligo, type 1 diabetes, non-obese diabetes, myasthenia gravis, Grave's disease, Hashimoto's thyroiditis, sclerosing cholangitis, sclerosing sialadenitis, systemic lupus erythematosis, autoimmune thrombocytopenia purpura, Goodpasture's syndrome, Addison's disease, systemic sclerosis, polymyositis,dermatomyositis, autoimmune hemolytic anemia, pernicious anemia, and the like.

In another embodiment, the method is directed to treating inflammatory diseases. "Inflammation" or an "inflammatory process" refers to a complex series of events, including dilatation of arterioles, capillaries and venules, with increased permeability and blood flow, exudation of fluids, including plasma proteins and leukocyte migration into the inflammatory focus. Inflammation may be measured by many methods well known in the art, such as the number of leukocytes, the number of polymorphonuclear neutrophils (PMN), a measure of the degree of PMN activation, such as luminal enhanced-chemiluminescence, or a measure of the amount of cytokines present.

An "immunosuppressive agent" to a pharmaceutically active agent that can decrease an immune response such as an inflammatory reaction. Immunosuppressive agents include, but are not limited to an agent of use in treating arthritis (anti-arthritis agent). Specific, non-limiting examples of immunosuppressive agents are non-steroidal anti-inflammatory agents, cyclosporine A, FK506, and anti- CD4. Rapamycin is an additional example of an immunosuppressive agent.

In one embodiment, the methods are provided for eliminating vascular obstructions.

In one embodiment, the method comprises inserting an implantable medical device in the form of vascular stent into a blood vessel, the stent having a generally tubular structure, the surface of the structure being coated with a composition as described above, such that the vascular obstruction is eliminated. For example, stents may be placed in a wide array of blood vessels, both arteries and veins, to prevent recurrent stenosis (restenosis) at, e.g., a site of (failed) angioplasties, to treat narrowings that would likely fail if treated with angioplasty, and to treat post surgical narrowings (e.g., dialysis graft stenosis).

Representative examples of suitable sites to be treated in the methods of the invention include, e.g., the iliac, renal, and coronary arteries, the superior vena cava, and in dialysis grafts. Within one embodiment, angiography is first performed in order to localize the site for placement of the stent. This is typically accomplished by injecting radiopaque contrast through a catheter inserted into an artery or vein as an x-ray is taken. A catheter may then be inserted either percutaneously or by surgery into the femoral artery, brachial artery, femoral vein, or brachial vein, and advanced into the appropriate blood vessel by steering it through the vascular system under fluoroscopic guidance. A stent may then be positioned across the vascular stenosis. A post insertion angiogram may also be utilized in order to confirm appropriate positioning.

Another embodiment of the invention includes a use as defined above wherein the medicament comprises a further therapeutic agent as defined above. In one embodiment, the further therapeutic agent is selected from antiproliferative, antimitotic, antimicrobial, anticoagulant, fibrinolytic, anti-inflammatory, immunosuppressive, and anti-antigenic agents. In another embodiment, the pharmaceutically active agent is agent is paclitaxel, a derivative of paclitaxel, sirolimus (rapamycin), and a derivative of sirolimus.

One embodiment provides a coated stents with heparinized polymer in combination with an antiproliferative drugs such as sirolimus, Paclitaxel or another selected active drug (e.g. a flavonoid or a derivative or analog of one or more of the foregoing named drugs) to reduce platelet adhesion and SMC (smooth muscle cell) proliferation simultaneously. Combining anti-coagulants with anti-proliferative agents to prevent the root causes of restenosis can resolve a renarrowing of treated arteries. In a heparinized polymeric matrix as a stent coating together with a selected antiproliferative drug blended into the polymer matrix, both drugs can release sufficiently simultaneously to arterial cells, which can result in inhibiting platelet adhesion and excess SMC growth.

In one embodiment, the heparinized biodegradable polymer is effectively miscible enough with the select drugs (e.g. paclitaxel, sirolimus (rapamycin), flavonoids such as genistein) that the drugs can be dispersed throughout the matrix of the heparinized polymer in a concentration sufficient to impart the ability of the polymer matrix to elute the drug over a long enough period of time from the date of first implant, e.g. 30-120 days, to prevent restenosis, thrombosis and/or to reduce platelet adhesion and SMC (smooth muscle cell) proliferation simultaneously.

In one embodiment, the pharmaceutically active agent is nonpolar relative to a heparinized biodegradable polymer. The miscibility of a nonpolar compound with a heparinized biodegradable polymer result may be surprising because the heparin moiety is polar and inherently immiscible with relatively non-polar moieties such as sirolimus, paclitaxel, (and related analogs and derivatives such as tacrolimus, zotarolimus, everolimus, and flavonoids such as genistein. It is believed that the covalent bonding of heparin to the biodegradable polymer creates a new biodegradable polymer substrate that is fundamentally different in structure and reactivity from the original biodegradable polymer such that in vivo release of heparin over the full term of in vivo degradation of the biodegradable polymer is accomplished simultaneously with normal non-covalently bound elution of the selected drug that is dispersed throughout the matrix of the polymer substrate.

### Assays

The stents disclosed herein can be tested by any number of methods known in the art. For example, assays for testing stents include assaying the release of drugs from polymer coated stents, their blood compatibility, their potential to cause inflammation, and their efficacy in preventing diseases, such as restenosis, in animal models.

An exemplary assay for release of drugs in-vitro from polymer coated stents is described in U.S. Patent No. 6,702,850, Example 8, in which the disclosure of Example 8 is incorporated herein by reference. The elution of a drug, such as paclitaxel from single or multilayer polymer coated stainless steel samples can be measured by incubating the samples in a buffer solution at 37°C, extracting each sample with a solvent, and determining the amount of paclitaxel eluted by HPLC.

An exemplary assay for blood compatibility of polymer coated stents is described in U.S. Patent No. 6,702,850, Example 5, in which the disclosure of Example 5 is incorporated herein by reference. The blood compatibility of a stent can be determined with a whole blood test, where the stents can be dipped in blood, such as fresh rabbit blood, and subsequently examined for the level of thrombus (clot) formation. As a control, a bare metal stent can provide a relatively high level of blood coagulation (thrombus formation) on its surface. A stent as disclosed herein should display less thrombus formation.

An exemplary assay for blood compatibility of polymer coated stents is described in U.S. Patent No. 6,702,850, Example 6, in which the disclosure of Example 6 is incorporated herein by reference. The blood compatibility of a multiple layer coated stent can be assessed with a platelet adhesion test, in which stents can be incubated in platelets in plasma isolated from fresh rabbit blood. After washing the stents with buffer, fixing with glutaraldehyde, and dehydrating with ethanol, the stents can be assessed by scanning electron microscopy to determine the platelet concentration on the stent surface. A bare metal stent should show a relatively uniform distribution of platelet adhesion. A stent as disclosed herein should display a decrease in platelet adhesion.

An exemplary assay for inflammation caused by polymer coated stents is described in U.S. Patent No. 6,702,850, Example 7, in which the disclosure of Example 7 is incorporated herein by reference. Evaluating inflammation of a stent as disclosed herein can be performed by implanting the stainless steel strips coated with the compositions disclosed herein into the backs of rats as well as bare stainless steel strips. The strips and surrounding tissue can be recovered after at least two weeks and less than one month and examined for inflammation. Strips coated with the compositions disclosed herein should cause less inflammation compared to bare strips.

An exemplary assay for determining the efficacy of the stents disclosed herein in treating restenosis can be performed in animal models such as a pig model, as described in U.S. Patent No. 6,702,850, Examples 11-13, in which the disclosure of Examples 11-13 are incorporated herein by reference. Stainless steel stents coated with the compositions disclosed herein can be inserted into coronary arteries of pigs via the carotid artery using a guide wire and a balloon catheter. The balloon can be inflated to its maximum size to intentionally damage the artery. After approximately one month, the damaged artery can be removed from the euthanized pigs and evaluated for neointima formation by light microscopy of fixed tissue sections. Before euthanasia the arteries can be evaluated by coronary angiography for size and narrowing compared to the same artery before the injury. The arteries implanted with stents disclosed herein can be compared with other animals implanted with bare stents or stents coated with polymer only. The stents disclosed herein should show a reduced neointima formation compared with the control stents.

An exemplary assay for determining the efficacy of restenosis via an animal model can involve assessing the therapeutic effect of a vascular injury, as described in EP1258258, Comparative Example 3 and Evaluation Test 1, the disclosures of which are incorporated herein by reference. Rabbit iliac arteries can be abraded with a balloon introduced through the femoral artery. A stent coated with the compositions disclosed herein can be implanted into rabbits fed for two weeks with a diet containing 1 % cholesterol additive by inserting the stent into the right iliac artery via the carotid artery using a guidewire and a balloon catheter, after abrading the artery with the balloon. A stainless steel stent coated with polymer only can be inserted into the left iliac artery. The rabbits can be fed with 0.5 % cholesterol additive for 4 weeks. After euthanizing and fixing the target blood vessel, the vessel can be sectioned and stained for light microscopic evaluation of the thickness of intimal hyperplasia. The stents disclosed herein should show a reduced intimal hyperplasia compared with a stent coated with polymer only.

Another animal model assay for restenosis can involve assessing the treatment of neointima formation following vascular injury, as disclosed in Jaschke et al., FASEB J. 2004 Aug;18(11):1285-7, which describes local cyclin-dependent kinase inhibition by inhibiting coronary artery smooth muscle cell proliferation and migration. Rat carotid arteries can be injured by withdrawal of an inflated balloon. Stents as disclosed herein and uncoated stents can be inserted into the injured arteries. After euthanizing the rats after 14 days, the carotids can be fixed, sectioned and stained for evaluation. The stents disclosed herein should show reduced neointima formation compared to uncoated stents.

### RELATED APPLICATIONS

This application claims the benefit of priority to U.S. Provisional Patent Application Serial No. 60/862,270 filed October 20, 2006, entitled "Coatings For Implantable Medical Devices"; U.S. Provisional Patent Application Serial No. 60/862,265 filed October 20, 2006, entitled "Compositions Comprising Porous Articles And Uses In Implantable Medical Devices"; U.S. Provisional Patent Application Serial No. 60/862,263 filed October 20, 2006, entitled "Compositions and Coatings For Implantable Medical Devices"; U.S. Provisional Patent Application Serial No. 60/832,383 filed July 21, 2006, entitled "Drug Coated and Releasing Balloon Catheters"; U.S. Provisional Patent Application Serial No. 60/814,973 filed June 20, 2006, entitled "Drug Eluting Stent"; and U.S. Provisional Patent Application Serial No. 60/780,121 filed March 8, 2006, entitled "Drug Eluting Stent", the disclosures of all of the foregoing of which are incorporated herein by reference.

### EXAMPLES

### Example 1: Manufacture of Drug Eluting Stent

The stents were manufactured from surgical grade Stainless Steel 316 L tube. Tubes were first cut with a laser machine according to a programmed design. The cut stents were electropolished for surface smoothness. The polished stents were then transferred to a clean room for a quality check. In a coating room, the stents were coated with paclitaxel. The coated stents were crimped on rapid exchange balloon catheters. The packed stents were sterilized with EtOH. A quality check was carried out at each and every stage and non-conforming stents were rejected.

### Example 2: Preparation of Heparinized Poly-L-Lactide (PLLA)

The synthesis of a heparinized poly-1-lactide is outlined below.

### Materials

1) poly-1-lactide (inherent viscosity = 2.6 - 3.2 dL/g)
2) heparin sodium (from porcine intestinal mucosa, 150-190 IU/mg)
3) dicyclohexylcarbodiimide (DCC)
4) 4-(dimethyl amino) pyridine (DMAP)
5) formamide
6) *N,N*-dimethyl formamide (DMF)

### Method

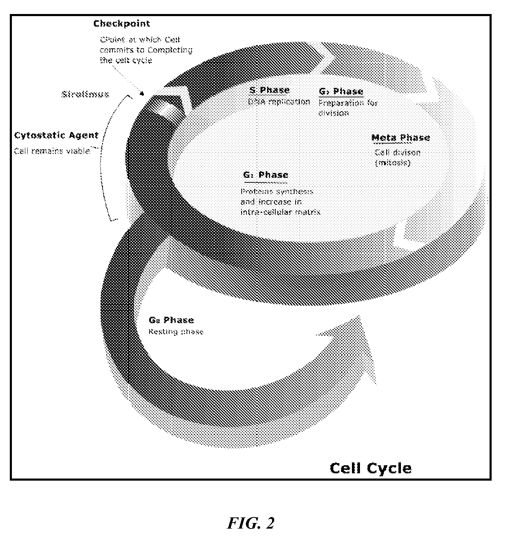

Heparin-conjugated PLLA was prepared by a direct coupling reaction using dicyclohexylcarbodiimide (DCC) / 4-(dimethyl amino) pyridine (DMAP). The experimental set-up is depicted in Fig 2.

Heparin (0.6 g, 1x10⁻⁴ mol) and PLA (6.0 g, 0.5x10⁻⁴ mol) were first dissolved in the *N,N*-dimethyl formamide (250 ml) and dichloromethane (DCM, 500 ml), respectively. The heparin solution was stirred and heated in a round bottom flask for 1 hr at a temperature of 50-55°C. Solutions of DCC (0.02 ml 0.1 M) and DMAP (0.2 ml 1.0 M) were then added to the heparin solution followed by addition of the PLGA solution dropwise over a time period of 15 minutes by pipette. Nitrogen gas was purged through the solution to create an inert atmosphere. The temperature of the reaction mixture was maintained at 50°C for 12 hrs.

The reaction mixture was then transferred to a 2000 ml beaker. Addition of methanol (1100 ml) caused the formation of precipitates, which were then filtered through Whatman filter paper (paper no. 42 - 2.5µm). After dissolving the precipitate in chloroform (600 ml), 500 ml deionized water was added to remove un-reacted Heparin. The organic layer was separated with a separatory funnel, and the washing with water was repeated five times. The product was then re-precipitated by the addition of excess methanol (1800 ml) to the organic layer. The mixture was cooled to 0-5°C for 12 hours. The final precipitate was collected by filtration and subjected to drying at 35°C for 12 hrs under vacuum. The competition of reaction is confirmed by conducting Fourier Transform Infrared (FT-IR) using KBr pellet method.

### Example 3: Preparation of Heparinized 50/50 Poly-D,L-Lactide-co-Glycolide

The synthesis of a heparinized 50/50 poly-d,1-lactide-co-glycolide is outlined below.

### Materials

1) 50/50 Poly L-Lactide-co-Glycolide (PLGA)
2) heparin sodium (from porcine intestinal mucosa, 150 -190 IU/mg)
3) dicyclohexylcarbodiimide (DCC)
4) 4-(dimethyl amino) pyridine (DMAP)
5) *N,N*-dimethyl formamide (DMF)

### Method

Heparin-conjugated PLGA was prepared by a direct coupling reaction using dicyclohexylcarbodiimide (DCC) / 4-(dimethyl amino) pyridine (DMAP) chemistry with an experimental set-up as described in Example 2.

Heparin (0.6 g, 1x10⁻⁴ mol) and PLGA (6.0 g, 0.5x10⁻⁴ mol) were first dissolved in the *N,N-*dimethyl formamide (250 ml) and dichloromethane (DCM, 500 ml), respectively. The heparin solution was stirred and heated in a round bottom flask for 1 hr at a temperature of 50-55°C. Solutions of DCC (0.05 ml 0.1 M) and DMAP (0.5 ml 1.0 M) were then added to the heparin solution followed by addition of the PLGA solution dropwise over a time period of 15 minutes by pipette. Nitrogen gas was purged through the solution to create an inert atmosphere. The temperature of the reaction mixture was maintained at 50°C for 12 hrs. The reaction mixture was then cooled to room temperature.

The reaction mixture was then mixed with a CHCl₃:H₂O mixture in a 1:1.5:2.5 ratio, followed by vigorous mixing for 10 min. The mixture was allowed to separate in two layers, where each layer was separated with a separating funnel. The chloroform layer was washed with an equal amount of water under vigorous shaking and subsequent separation of the chloroform layer. The CHC1₃ layer was evaporated using a rotary evaporator to a minimal amount (≤1/10^{th}), followed by mixing with 1.5 times the amount of water to extract the concentrate. Methanol equivalent to 3X ml of the extract concentrate was added. This mixture was shaken to precipitate Hep-PLGA. The precipitate was isolated by filtration and by scraping the Hep-PLGA from the vessel surface. The precipitate was then dissolved in a minimum amount of CHCl₃ and re precipitated by adding water and methanol. The final product was dried at 40-50° C and stored in an air tight container at low temperature as per storage condition of parent polymer. The completion of reaction is confirmed by FT-IR using KBr pellet.

### Example 4: Preparation of Heparinized 70/30 Poly-L-Lactide-co-Caprolactone

The synthesis of a heparinized 70/30 poly-1-lactide-co-caprolactone is outlined below.

### Materials

1) poly-1-lactide-co-caprolactone (PLC)
2) heparin sodium (from porcine intestinal mucosa, 150 -190 IU/mg)
3) dicyclohexylcarbodiimide (DCC)
4) 4-(dimethyl amino) pyridine (DMAP)
5) *N,N*-dimethyl formamide (DMF)

### Method

Heparin-conjugated PLGA was prepared by a direct coupling reaction using dicyclohexylcarbodiimide (DCC) / 4-(dimethyl amino) pyridine (DMAP) chemistry with an experimental set-up as described in Example 2.

Heparin (0.6 g, 1x10⁻⁴ mol) and PLC (6.0 g, 0.5x10⁻⁴ mol) were first dissolved in the *N,N-*dimethyl formamide (250 ml) and dichloromethane (DCM, 500 ml), respectively. The heparin solution was stirred and heated in a round bottom flask for 1 hr at a temperature of 50-55°C. Solutions of DCC (0.02 ml 0.1 M) and DMAP (0.2 ml 1.0 M) were then added to the heparin solution followed by addition of the PLGA solution dropwise over a time period of 15 minutes by pipette. Nitrogen gas was purged through the solution to create an inert atmosphere. The temperature of the reaction mixture was maintained at 50°C for 12 hrs.

The reaction mixture was then transferred to a 2000 ml beaker. Addition of methanol (1100 ml) caused the formation of precipitates, which were then filtered through Whatman filter paper (paper no. 42 - 2.5µm). After dissolving the precipitate in chloroform (600 ml), 500 ml deionized water was added to remove un-reacted Heparin. The organic layer was separated with a separatory funnel, and the washing with water was repeated five times. The product was then re-precipitated by the addition of excess methanol (1800 ml) to the organic layer. The mixture was cooled to 0-5°C for 12 hours. The final precipitate was collected by filtration and subjected to drying at 35°C for 12 hrs under vacuum. The competition of reaction is confirmed by conducting Fourier Transform Infrared (FT-IR) using KBr pellet method.

### Example 5: Preparation of Heparinized Polyvinylpyrrolidone

The synthesis of a heparinized polyvinylpyrrolidone is outlined below.

### Materials

1) polyvinylpyrrolidone (PVP)
2) heparin sodium (from porcine intestinal mucosa, 150 -190 IU/mg)
3) chloroform
4) pyridine
5) thionyl chloride
6) *N,N*-dimethyl formamide (DMF)

### Method

The preparation of heparinized polyvinylpyrrolidone was divided into two steps:

*1) Activation of Poly-N-Vinyl Poly Pyrrolidone (Formation of Imidoyl Ion of PVP).* In a three-neck 500 ml round bottom flask equipped with a West condenser (air cooled) connected to a U-shaped Drierite drying tube, a pressure equalized dropping funnel and a glass stopper, a solution of polyvinylpyrrolidone (PVP) was stirred vigorously in 100 ml of chloroform and 20 ml of pyridine. Redistilled thionyl chloride (5 ml, 8.3 g, 0.119 mole) in 40 ml of chloroform was added dropwise to the PVP solution. The rate of addition was such that a gentle reflux of the reaction mixture was maintained. The addition lasted about 1/2 hour, and the solution changed from light yellow to clear dark reddish brown. The reaction solution was stirred and allowed to cool to room temperature for about 2 hours. The imidoyl ions of PVP were formed in chloroform/pyridine solution.

*2) Reaction of Heparin with Imidoyl Ions of PVP in Heterogeneous Medium.* Heparin in 75 ml of 20% Na₂CO₃ solution was added by stirring to imidoyl ions of PVP in chloroform-pyridine solution in which PVP was activated by a slight excess of thionyl chloride (5.0 ml or 8.3 g, 0.119 mole). The heparin addition lasted 30 minutes, during which heat and CO₂ evolved. The reaction mixture was stirred and allowed to cool down to room temperature. Then the organic (chloroform-pyridine) layer of the solution mixture was separated from the aqueous layer. Evaporation of the organic layer under reduced pressure gave a residue with a trace of bitter odor (pyridine). The residue re-dissolved in a minimum of water. To the aqueous solution of the residue from the organic layer as well as to the aqueous layer from the reaction mixture, 50 ml of 5% cetyl pyridinium chloride (CPC) solution was added. White precipitates formed immediately. The precipitates were then filtered. A small additional amount of 5% CPC solution was added to each of the filtrates to check the completion of CPC precipitation. Repetitive precipitations were needed for the filtrate obtained from the aqueous layer. PVP-heparin was recovered by dissolving the white CPC complex in 3.2 N MgCl₂, adding 50 ml of 2.5 N potassium thiocyanate to each of the solutions to precipitate CPC. Filtering the suspensions, the filtrate was dialyzed extensively against water as follows: 4 hours, 2 x 10 1; 24 hours, 2 x 10 1. Lyophilization of the dialysates gave white powders of 0.79 g from organic layer (H-I) and 6.35 g (H-II) from the aqueous layer, both gave positive Toluidine blue tests and positive tests with chloroform-iodine solution. The competition of reaction was confirmed by conducting Fourier Transform Infrared (FT-IR) and NMR spectroscopy.

### Example 6: Characterization of PLLA-Heparin conjugate

1. The molecular weight of PLLA is measured by gel permeation chromatography (GPC).

2. The structure of PLLA-heparin is characterized by a Fourier Transform Infrared Spectrophotometer (FT-IR).

3. The content of conjugated heparin can be analyzed by HPLC and toluidine blue colorimetric analysis using UV spectrophotometry operating at 631 nm.

### Example 7: General Conditions for the Manufacture of a Drug Eluting Stent

The Example illustrates the preparation of stents containing multiple layers. One of ordinary skill in the art can readily manufacture a stent containing one or more layers based on the teachings of this Example.

Multilayered stents can be manufactured from solutions of different polymers optionally containing therapeutic agents. The top coating can be a protective layer, such as a solution of heparinized polyvinylpyrrolidone in dichloromethane. The final stent can contain four layers by respectively spraying four separate solutions.

The coating process was performed in aseptic conditions under controlled environment and clean room conditions. The temperature and humidity were maintained at 23±3°C and 60±5 % Rh respectively in a clean room. The drug coating machine parameters were checked and set according to the predetermined stent size. The spray gun angle, distance between spray gun tip and stent and alignment of machine were also checked and set. If necessary, the gun was cleaned with a solvent such as dichloromethane (DCM) before starting the coating process.

The heparinized polymer and pharmaceutically active agents were provided as solutions or suspensions. The concentration of drugs were mixed together with the heparinized polymer depending on the selection of drug or drugs to be loaded on a stent or other delivery device.

The stent can be hung between two collate with the help of hooks. The first solution or suspension can be sprayed on the stent at an optimum flow rate under the necessary amount of nitrogen pressure. During coating, the flow rate is maintained. The coating layer is dried for 10 minutes prior to spraying the next layer. Subsequent layer coatings can be performed in the same manner. The final layer was added by spraying the solution of heparinized polyvinylpyrrolidone in dichloromethane.

After removing the stent from collate and completion of the layer coating, the weight of the stent is measured and recorded. The surface of the stent is checked under a microscope. The drug coated stents are kept in an air tight centrifuge tube and transferred to the clean room for further processing.

### Example 8. Stent coated with porous poly-L-lactic acid (solid particle porogen method)

Solid ascorbic acid is micronized to a range of particle sizes of < 1 micron. Poly-l-lactic acid is dissolved in dichloromethane. Ascorbic acid (porogen) and the poly-l-lactic acid solution are mixed and placed on a rolling glass ball mill until the solid is evenly dispersed. The suspension is sprayed onto stents by the method described in Example 7 and the coating is dried under vacuum. The coated stents are soaked in methanol until all the ascorbic acid is dissolved leaving pores of the desired size. The stents are again dried under vacuum. Heparinized poly-L-lactic acid prepared from Example 2 are formed into nanoparticles containing a dispersed therapeutic agent (paclitaxel, rapamycin, genistein, flavonoid , LY294002 or LY303511) as prepared by methods known in the art (for example, see Example 8 of U.S. Patent No. 5,716,981, in which the disclosure of Example 8 is incorporated herein by reference). The nanoparticles are isolated by centrifugation, suspended in water and sprayed onto the stent. The stent is dried and spray-coated with polyvinylpyrrolidone dissolved in dichloromethane as a protective layer in a manner similar to that described in Example 7.

### Example 9. Stent coated with porous heparinized poly-L-lactic acid and polyvinylpyrrolidone (solid particle porogen method)

Solid glucose is micronized to a range of particle sizes of < 1 micron, suspended in a mixture of heparinized poly-L-lactide and polyvinylpyrrolidone dissolved in dichloromethane, mixed and sprayed onto stents as described in Example 7. The stents are soaked in water until the glucose dissolves leaving pores of the desired size. Nanoparticles (as described in Example 7) of a mixture of heparinized poly-1-lactide (as described in Example 2), polyvinylpyrrolidone and genistein are deposited in the pores and the dried stents are top-coated with heparinized polyvinylpyrrolidone as in Example 7.

### Example 10. Stent coated with a mixture of heparinized poly-l-lactide, heparinized 50/50 poly-D,L-lactide-co-glycolide and heparinized polyvinylpyrrolidone (solid particle porogen method)

Solid sucrose is micronized to a range of particle sizes of < 1 micron, suspended in a mixture of heparinized poly-L-lactide (Example 2), heparinized 50/50 poly-d,l-lactide-co-glycolide (Example 3) and heparinized polyvinylpyrrolidone (Example 5) dissolved in dichloromethane, mixed and sprayed onto stents as described in Example 7. The stents are soaked in water until the sucrose dissolves leaving pores of the desired size. Nanoparticles of a mixture of heparinized poly-L-lactide, heparinized 50/50 poly-d,l-lactide-co-glycolide, heparinized polyvinylpyrrolidone and a mixture of genistein and sirolimus are deposited in the pores and the dried stents are top-coated with heparinized polyvinylpyrrolidone as in Example 7.

### Example 11. Stent coated with a mixture of heparinized 70/30 poly-l-lactide-co caprolactone, heparinized 50/50 poly-D,L-lactide-co-glycolide and heparinized polyvinylpyrrolidone (solid particle porogen method)

Solid mannitol is micronized to a range of particle sizes of < 1 micron, suspended in a mixture of heparinized 70/30 poly-1-lactide-co-caprolactone (Example 4), heparinized 50/50 poly-d,1-lactide-co-glycolide (Example 3) and heparinized polyvinylpyrrolidone (Example 5) dissolved in dichloromethane, mixed and sprayed onto stents as described in Example 7. Nanoparticles of a mixture of 70/30 poly-1-lactide-co caprolactone, heparinized 50/50 poly-d,1-lactide-co-glycolide, heparinized polyvinylpyrrolidone and a mixture of genistein and paclitaxel are deposited in the pores and the dried stents are top-coated with heparinized polyvinylpyrrolidone as in Example 7.

### Example 12. Stent Coated with Porous Poly-L-Lactic Acid (TIPS method by decreasing the temperature)

The TIPS method used here is that of YS Nam and TG Park, 1999, Porous biodegradable polymeric scaffolds prepared by thermally induced phase separation, J Biomed Mater Res 47: 8-17. Poly-L-lactic acid (100 mg) is dissolved in 5 ml of a mixture of dioxane and water at a ratio of from 84:16 to 90: 10 v/v and warmed above the cloud point. The clear homogeneous mixture is sprayed onto stents in an atmosphere saturated with the same mixture of dioxane and water at the same temperature. The liquid coated stent is then rapidly cooled to -40 °C, held at this temperature overnight, and then freeze-dried for 3 days. The stent is then sprayed with heparinized polymer nanoparticles containing genistein and top-coated with polyvinylpyrrolidone as in Example 7.

### Example 13. Stent Coated with Porous Heparinized Poly-L-Lactic Acid (TIPS method by increasing the temperature)

Heparinized PLLA is dissolved in dichloromethane. The clear homogeneous mixture is sprayed onto stents using ultrasonic atomization technique at lower temperatures, as can be determined by one of ordinary skill in the art. The coated stent is then kept in vacuum oven at below glass transition temperature of the polymers for 2 hours. The pore size can be controlled in the range from 0.1 to 50 micron by changing the evaporation rate of the polymer solution. The stent is then sprayed with sirolimus loaded heparinized polymeric nanoparticles and coated with top layer of polyvinylpyrrolidone as in Example 7.

### Example 14. Stent coated with porous heparinized poly-L-lactide and polyinylpyrrolidone (TIPS method by increasing the temperature)

Starting with a mixture of heparinized poly-L-lactic acid and polyvinylpyrrolidone and following the procedure of Example 13, a stent coated with porous heparinized poly-L-lactic acid and polyvinylpyrrolidone is prepared. This stent is sprayed with nanoparticles of a mixture of heparinized poly-L-lactide, heparinized 50/50 poly-d,1-lactide-co-glycolide, heparinized polyvinylpyrrolidone and genistein and then top-coated as described in Example 7.

### Example 15. Stent coated with a mixture of heparinized poly-L-lactide, heparinized 50/50 poly-D,L-lactide-co-glycolide and heparinized polyvinylpyrrolidone (TIPS method by increasing the temperature)

Starting with a mixture of heparinized poly-L-lactide, heparinized 50/50 poly-d,l-lactide-co-glycolide and heparinized polyvinylpyrrolidone and following the procedure of Example 13, a stent coated with porous heparinized poly-L-lactide, heparinized 50/50 poly-d,1-lactide-co-glycolide and heparinized polyvinylpyrrolidone is prepared. This stent is sprayed with nanoparticles of a mixture of heparinized poly-L-lactide, heparinized 50/50 poly-d,1-lactide-co-glycolide, heparinized polyvinylpyrrolidone, genistein and sirolimus and then top-coated as in Example 7.

### Example 16. Stent coated with a mixture of heparinized 70/30 poly-L-lactide-co caprolactone, heparinized 50/50 poly-D,L-lactide-co-glycolide and heparinized polyvinylpyrrolidone (TIPS method by increasing the temperature)

Starting with a mixture of heparinized 70/30 poly-L-lactide-co-caprolactone, heparinized 50/50 poly-d,l-lactide-co-glycolide and heparinized polyvinylpyrrolidone and following the procedure of Example 13, a stent coated with this polymer mixture is prepared. This stent is sprayed with nanoparticles of a mixture of heparinized 70/30 poly-L-lactide-co-caprolactone, heparinized 50/50 poly-d,1-lactide-co-glycolide, heparinized polyvinylpyrrolidone and a mixture of genistein and paclitaxel and then top-coated as described in Example 7.

## Claims

1. A composition comprising:
a first polymer having pores;
nanoparticles dispersed within the pores of the first polymer, the nanoparticles comprising a second polymer and at least one pharmaceutically active agent dispersed in the second polymer; and
heparin covalently bonded to at least one of the first and second polymer.

2. The composition of claim 1, wherein the heparin is covalently bonded to one or both of the first and second polymers.

3. The composition of claim 1, wherein the polymer covalently bonded to heparin is biodegradable.

4. The composition of claim 1, wherein one or both of the first and second polymers is biodegradable.

5. The composition of claim 4, wherein the biodegradable polymer is chosen from poly(1-lactide), racemic polylactide, poly(1-lactide-co-glycolide), racemic poly(1-lactide-co-glycolide), poly(1-lactide-co-caprolactone poly(d,l-lactide-co-caprolactone), poly(l-lactide-co-trimethylene carbonate) and poly(d,1-lactide-co-trimethylene carbonate).

6. The composition of claim 4, wherein the biodegradable polymer is chosen from polylactides.

7. The composition of claim 1, wherein the first and second polymers are the same.

8. The composition of claim 1, wherein at least one of the first and second polymers comprises a blend of one or more polymers.

9. A device comprising a coating for at least a portion thereof, the coating comprising a composition comprising:
a first polymer having pores;
nanoparticles dispersed within the pores of the first polymer, the nanoparticles comprising a second polymer and at least one pharmaceutically active agent dispersed in the second polymer; and
heparin covalently bonded to at least one of the first and second polymers.

10. The device of claim 9, wherein the device is selected from sutures, staples, anastomosis devices, vertebral disks, bone pins, suture anchors, hemostatic barriers, clamps, screws, plates, clips, vascular implants, tissue scaffolds, bone substitutes, intraluminal devices, and vascular supports.

11. The device of claim 9, wherein the device is implantable into a mammalian lumen.

12. The device of claim 9, wherein the device is a stent.

13. The device of claim 12, wherein the concentration of the at least one pharmaceutically active agent based on the surface area of the stent ranges from 0.1 to about 5 µg/mm².

14. The device of claim 12, wherein the concentration of the at least one pharmaceutically active agent based on the surface area of the stent ranges from about 0.7 µg/mm² to about 3.0 µg/mm²

15. The device of claim 12, wherein the concentration of the at least one pharmaceutically active agent based on the surface area of the stent ranges from about 1.0 to about 1.8 µg/mm²

16. The device of claim 12, wherein the concentration of the at least one pharmaceutically active agent based on the surface area of the stent ranges from about 1.0 to about 1.4 µg/mm².

17. The device of claim 9, wherein the coating contacts the medical device.

18. The device of claim 9, wherein the coating contacts at least one inner coating that contacts the medical device.

19. The device of claim 18, wherein the at least one inner coating is chosen from ceramics, nonbiodegradable polymers, metals, and carbon.

20. The device of claim 9, wherein the device further comprises a protective coating over the coating, wherein the protective coating is free of a pharmaceutically active agent.

21. The device of claim 20, wherein the protective coating comprises at least one biodegradable polymer.

22. The device of claim 9, wherein the at least one biodegradable polymer is covalently bonded to heparin.

23. The device of claim 9, wherein the device further comprises at least one additional coating comprising a polymer covalently bonded to heparin, the at least one additional coating comprising at least one pharmaceutically active agent.

24. The device of claim 23, wherein the polymer is biodegradable.

25. The device of claim 23, wherein the device comprises at least two additional coatings.

26. The device of claim 23, wherein the device comprises at least one additional coating and a protective coating.

27. The device of claim 9, wherein at least one of the first and second polymers degrades by hydrolysis in a natural intraluminal human body environment at preselected rates of degradation.

28. The device of claim 9, wherein the at least one pharmaceutically active agent is chosen from antithrombotics, anticoagulants, antiplatelet agents, thrombolytics, antiproliferatives, anti-inflammatories, antimitotic, antimicrobial, agents that inhibit restenosis, smooth muscle cell inhibitors, antibiotics, fibrinolytic, immunosuppressive, and anti-antigenic agents.

29. The device of claim 9, wherein the at least one pharmaceutically active agent is chosen from paclitaxel, sirolimus, flavonoids, compounds of formula A 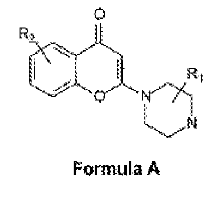 wherein R1 and R2 are each independently selected from alkyl, substituted alkyl, heteroalkyl, cycloalkyl, substituted cycloalkyl, heterocycloalkyl, substituted heterocycloalkyl, aryl, substituted aryl, heteroaryl, substituted heteroaryl, alkoxy, halogen, hydroxy, and amino,
and compounds of formula B, 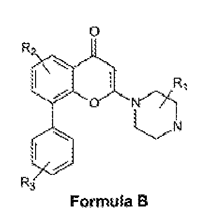 wherein the presence of each of R1 and R2 and R3 is optional and R1 and R2 and R3 are each independently selected from alkyl, aryl, alkoxy, halogen, hydroxy, and amino.

30. The device of claim 29, wherein the flavonoid is selected from chalcones, dihydrochalcones, flavanones, flavonols, dihydroflavonols, flavones, flavanols, isoflavones, neoflavones, aurones, anthocyanidins, proanthocyanidins and isoflavanes.

31. The device of claim 29, wherein the flavonoid is selected from flavanones, flavonols, and isoflavones.

32. The device of claim 29, wherein the flavonoid is selected from narigenin, naringin, eriodictyol, hesperetin, hesperidin (esperidine), kampferol, quercetin, rutin, cyanidol, meciadonol, catechin, epi-gallocatechin-gallate, taxifolin (dihydroquercetin), genistein, genistin, daidzein, biochanin, glycitein, chrysin, diosmin, luetolin, apigenin, tangeritin and nobiletin.

33. The device of claim 29, wherein the at least one pharmaceutically active agent is paclitaxel.

34. The device of claim 29, wherein the at least one pharmaceutically active agent is sirolimus.

35. The device of claim 29, wherein the at least one pharmaceutically active agent is genistein.

36. A device comprising a coating, comprising:
a polymer having pores;
nanoparticles dispersed within the pores of the polymer, the nanoparticles comprising at least one pharmaceutically active agent;
wherein the at least one pharmaceutically active agent is chosen from composition comprising at least one pharmaceutically active agent selected from genistein or compounds having the structure of Formula A and Formula B below: 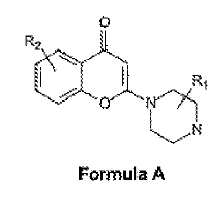 wherein R1 and R2 are each independently selected from alkyl, substituted alkyl, heteroalkyl, cycloalkyl, substituted cycloalkyl, heterocycloalkyl, substituted heterocycloalkyl, aryl, substituted aryl, heteroaryl, substituted heteroaryl, alkoxy, halogen, hydroxy, and amino, 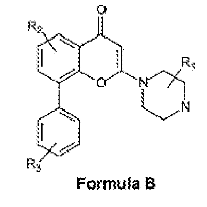 wherein the presence of each of R1 and R2 and R3 is optional and R1 and R2 and R3 are each independently selected from alkyl, aryl, alkoxy, halogen, hydroxy, and amino.

37. The device of claim 36, wherein the nanoparticles further comprise a polymer that is the same or different from the porous polymer, the nanoparticles being porous or nonporous.

38. The device of claim 36, wherein at least one of the nanoparticulate polymer and the porous polymer is biodegradable.

39. The device of claim 38, wherein the biodegradable polymer is chosen from poly(1-lactide), racemic polylactide, poly(1-lactide-co-glycolide), racemic poly(1-lactide-co-glycolide), poly(1-lactide-co-caprolactone poly(d,l-lactide-co-caprolactone), poly(1-lactide-co-trimethylene carbonate) and poly(d,l-lactide-co-trimethylene carbonate).

40. The device of claim 36, wherein at least one of the nanoparticulate polymer and the porous polymer further comprises heparin.

41. The device of claim 40, wherein the heparin is covalently bonded to the polymer.

42. The device of claim 36, wherein both the nanoparticulate polymer and the porous polymer comprise a biodegradable polymer covalently bound to heparin

43. A method of making an implantable medical device comprising:
coating the implantable medical device with a mixture comprising:
a polymer, polymer suspension, or polymer-containing solution, and
a solvent;
changing the temperature of the mixture to induce a phase separation of a polymer-rich phase and a polymer-poor phase; and
removing the solvent to form a porous polymer coating on the device.

44. The method of claim 43, wherein the method further comprises impregnating the pores of the polymer with nanoparticles comprising at least one pharmaceutically active agent.

45. The method of claim 44, wherein the nanoparticles further comprise a polymer which may be the same or different as the porous polymer.

46. The method of claim 45, wherein at least one of the porous polymer or the nanoparticulate polymer is covalently bound to heparin.

47. The method of claim 45, wherein at least one of the porous polymer or the nanoparticulate polymer is biodegradable.

48. The method of claim 45, wherein at least one of the porous polymer or the nanoparticulate polymer comprises one or more of poly(1-lactide), racemic polylactide, poly(l-lactide-co-glycolide), racemic poly(1-lactide-co-glycolide), poly(1-lactide-co-caprolactone poly(d,l-lactide-co-caprolactone), poly(l-lactide-co-trimethylene carbonate) and poly(d,l-lactide-co-trimethylene carbonate).

49. The method of claim 45, wherein both the nanoparticulate polymer and the porous polymer comprises a biodegradable polymer covalently bonded to heparin.

50. Use of a composition comprising:
a first polymer having pores;
nanoparticles dispersed within the pores of the first polymer, the nanoparticles comprising a second polymer and at least one pharmaceutically active agent dispersed in the second polymer; and
heparin covalently bonded to at least one of the first and second polymer;
for the manufacture of a medicament for treating at least one disease or condition associated with vascular injury or angioplasty, wherein the medicament is administered by implanting in a subject in need thereof a medical device having a coating for at least a portion thereof comprising the medicament.

51. A use according to claim 50, wherein the heparin is covalently bonded to both the first and second polymers.

52. A use according to claim 51, wherein the polymer covalently bonded to heparin is biodegradable.

53. A use according to claim 50, wherein one or both of the first and second polymers is biodegradable.

54. A use according to claim 53, wherein the biodegradable polymer is chosen from poly(l-lactide), racemic polylactide, poly(l-lactide-co-glycolide), racemic poly(l-lactide-co-glycolide), poly(1-lactide-co-caprolactone poly(d,l-lactide-co-caprolactone), poly(l-lactide-co-trimethylene carbonate) and poly(d,l-lactide-co-trimethylene carbonate).

55. A use according to claim 53, wherein the biodegradable polymer is chosen from polylactides.

56. A use according to claim 50, wherein the first and second polymers are the same.

57. A use according to claim 50, wherein at least one of the first and second polymers comprises a blend of one or more polymers.

58. A use according to claim 50, wherein the at least one disease or condition is a proliferative disorder.

59. A use according to claim 58, wherein the proliferative disorder is restenosis.

60. A use according to claim 58, wherein the proliferative disorder is a tumor.

61. A use according to claim 58, wherein the proliferative disorder comprises the proliferation of smooth muscle cells.

62. A use according to claim 58, wherein the at least one disease or condition is an inflammatory disease.

63. A use according to claim 58, wherein the at least one disease or condition is an autoimmune disease.

64. A use according to claim 58, wherein the at least one disease or condition is neointima and neointimal hyperplasia.

65. A use according to claim 58, wherein the at least one disease or condition is selected from thrombosis, embolism, and platelet accumulation.
